# EUROPEAN PATENT APPLICATION

(11) **EP 0 816 492 A1**
(43) Date of publication of application: **07.01.1998**
(21) Application number: 96941848.2
(22) Date of filing: 12.12.1996
(51) Int. Cl.: C12N 15/11, C07K 16/18, A61K 38/17, A61K 39/395

(54) **NOVEL DNAS, NOVEL POLYPEPTIDES AND NOVEL ANTIBODIES**

(30) Priority: 12.12.1995 JP 322745/95
(71) Applicant: Kyowa Hakko Kogyo Co., Ltd., Chiyoda-ku, Tokyo 100 (JP)
(72) Inventor: KUGA, Tetsuro, Tokyo 194 (JP); NAKAGAWA, Satoshi, Tokyo 194 (JP); SAKAKI, Yoshiyuki, Tokyo 108 (JP); ZHAO, Nanding, Tokyo 108 (JP); HASHIDA, Hideji, Tokyo 108 (JP)
(74) Representative: VOSSIUS & PARTNER
(86) International application number: JP9603630
(87) International publication number: WO9721807

(57) **Abstract**

A DNA selected from among those having the base sequences described in SEQ ID NOS: 1 to 17 and being complementary to the RNA strand of an mRNA the expression dose of which varies in the brain of a patient with Alzheimer's disease compared with the mRNA expressed in normal brain; a DNA fragment hybridizable with the above-mentioned DNA from which at least one base in the above DNA has been deleted or substituted by another base, or at least one base has been added to the above DNA; polypeptides encoded by these DNAs; antibodies recognizing these polypeptides; and diagnosis, treatment and studies on patients with Alzheimer's disease by using the DNAs, polypeptides and antibodies as described above.

## Description

### TECHNICAL FIELD

The present invention relates to DNAs each having a complementary sequence to the RNA strand of an mRNA expression level of which varies in the brain of a patient with Alzheimer's disease, compared with the mRNA expressed in the brain of normal human subjects, DNAs fragments hybridizable with such DNAs, polypeptides encoded by the DNAs and antibodies recognizing the polypeptides.

### BACKGROUND ART

It is estimated that about 100,000 genes are present in human chromosomes. The expression of these genes is controlled by a good-balanced regulation mechanism for a long term, whereby human bodies can be maintained healthy. More specifically, quantitative and qualitative disorders of the expression of the genes including for example the abnormal expression of the genes or the expression of abnormal gene products due to mutation cause a variety of diseases. If an abnormally expressed gene specific to a disease is identified, the gene and the protein encoded by the gene can be used for the diagnosis and therapeutic treatment of the disease.

The research works on the analysis of human genes are very meaningful not only for satisfying the interest from the field of basic biology but also for the development and research works of medicinal products and diagnostic agents. From such respect, the human genome projects for the elucidation of the entire nucleotide sequences of human genome are now under way worldwide.

However, even the currently top level scientific technology requires a long period of time for the elucidation of the whole structure of human genome, and therefore, a cDNA project to elucidate only the expressed genetic information has been drawing attention.

So far reported research works include, for example, reports about the determination of the partial sequences of cDNAs randomly selected from a cDNA library from human brain [Science 252, 1651 - 1656 (1991); Nature 355, 632 - 634 (1992); Nature Genetics 2, 180 - 185 (1992); Nature Genetics 4, 256 - 267 (1993); Nature Genetics 4, 373 - 380 (1993)], a report about the determination of the partial sequences of cDNAs randomly selected from a cDNA library from a human liver cell line [Nature Genetics 2, 173 - 179 (1992)], a report about the determination of the partial sequences of cDNAs randomly selected from a cDNA library of human islets of Langerhans [Human Molecular Genetics 2, 1793 - 1798 (1993)] and a report about the determination of the partial sequences of cDNAs randomly selected from a human keratinocyte cDNA library [Biochem. Biophys. Res. Communi. 202, 976 - 983 (1994)], or a report about the determination of the nucleotide sequence of a cDNA randomly selected from a cDNA livrary derived form a human cultivated cell line (WO 94/03599).

Because these reports have been attained with major attention focused on the determination of the nucleotide sequences, where the structures of these randomly selected genes have been elucidated, the functions of the products of these genes have simply been anticipated on the basis of the examination of the homology between unknown genes and known genes or proteins already analyzed and registered in the data base, although the information of the structures of these genes have been brought about. Thus, these reports cannot directly provide information from the respect of such an application as the development of pharmaceutical products.

### DISCLOSURE OF THE INVENTION

Focusing their attention to the fact that a gene expression level of which varies quantitatively or qualitatively in a patient with a disease has some function related to the pathological disorders, the present inventors have expected that simple and thorough screening of a cDNA which expression is abnormal in a patient with a disease to elucidate the structure thereof may serve well as a genetic analysis truly useful for the development and research works of the pharmaceutical products and the like of the disease. Therefore, the inventors have intensively progressed their works of the cDNA analysis. Consequently, the inventors have developed high-density cDNA filter analysis (referred to as "HDCFA" hereinafter) which enables the simple analysis of the expression levels of a vast amount of genes.

According to the method, mRNA with the increase or decrease in the expression level thereof in the brain of patients with Alzheimer's disease can be identified, compared with the expression level of the mRNA in normal human brain, and by recovering thereafter a DNA having a complementary sequence to the RNA strand of the mRNA, the present invention has been completed.

The present invention relates to DNAs each having a complementary sequence to the RNA strand of an mRNA expression of which is increased or decreased in the brain of patients with Alzheimer's disease, compared with the mRNA expressed in the brain of normal human subjects, DNAs hybridizable with such DNAs, polypeptides encoded by the DNAs, antibodies recognizing the polypeptides and the diagnosis, therapeutic treatment and research works of Alzheimer's patients using the DNAs, the polypeptides and the antibodies.

The present invention relates to DNAs complementary to the RNA strand of the mRNA, expression level of which varies in the brain of patients with Alzheimer's disease, compared with the mRNA expressed in the brain of normal human subjects, for example, a DNA having the nucleotide sequence selected from Sequence Nos. 1 to 17 and a DNA wherein one or more nucleotides in said DNA are deleted or substituted with other nucleotides or one or more nucleotides are added to said DNA, which is hybridizable with said DNA under stringent conditions. DNAs hybridizable under stringent conditions include a DNA with the nucleotide sequence 60 % or more, preferably 80 % or more and more preferably 95 % or more homologous with the nucleotide sequence of a DNA having the nucleotide sequence selected from Sequence Nos.1 to 17.

Herein, each of the DNAs having one of the nucleotide sequences of Sequence Nos.1 to 15 has a complementary sequence to the RNA strand of mRNA with the increase in the expression level in the brains of patients with Alzheimer's disease, and preferably, the increase in the expression level is 2-fold or more. Each of the DNAs having one of the nucleotide sequences of Sequence Nos.16 and 17 has a complementary sequence to the RNA strand of mRNA with the decrease in the expression level in the brain of patients with Alzheimer's disease, and preferably, the decrease in the expression level is 0.5-fold or less.

A DNA wherein one or more nucleotides in said DNA are deleted or substituted with other nucleotides or one or more nucleotides are added to said DNA can be generated by methods described in Nucleic Acids Research, 10, 6487 (1982), Proc. Natl. Acad. Sci., USA, 79, 6409 (1982), Proc. Natl. Acad. Sci., USA, 81, 5662(1984), Science, 224, 1431(1984), PCT WO 85/00817(1985), Nature, 316, 601 (1985), Gene, 34, 315 (1985), Nucleic Acids Research, 13, 4431(1985), Current Protocols in Molecular Biology, Chapter 8, Mutagenesis of Cloned DNA, John Wiley & Sons, Inc. (1985) and the like.

The term "DNA hybridizable under stringent conditions" means a DNA recovered by colony hybridization or plaque hybridization using as the probe a DNA complementary to the RNA strand of the mRNA with the expression level varied in the brain of Alzheimer's patients, compared with the expression level of the mRNA in normal human brain; more specifically, such DNA can be identified by the process comprising hybridizing in the presence of 0.7 to 1.0 M NaCl at 65 °C using a filter immobilized with the DNA derived from the colony or plaque and washing the filter using 0.1 x to 2 x SSC solutions (1 x SSC solution is composed of 150 mM sodium chloride and 15 mM sodium citrate) under a condition of 65 °C. The hybridization can be carried out according to the method described in Molecular Cloning, A Laboratory Manual, Second Edition, Sambrook, Fritsch & Maniatis, ed., Cold Spring Harbor Laboratory Press, 1989.

The polypeptides of the present invention are a polypeptides encoded by the DNAs, for example, a polypeptide encoded by a DNA having the nucleotide sequences selected from Sequence Nos. 1 to 17, or a polepeptide encoded by a DNA wherein one or more nucleotides in said DNA are deleted or substituted with other nucleotides or one or more nucleotides are added to said DNA, which is hybridizable with the DNA under stringent conditions.

The antibodies of the present invention include an antibody recognizing the polypeptide.

The DNAs of the present invention (referred to as "Alzheimer's disease-related DNA" hereinafter) are prepared by according to following steps;
1. preparing a human brain cDNA library;
2. preparing high-density cDNA filters using a cDNA derived from human brain;
3. preparing mRNAs from the brains of human patients with Alzheimer's disease and human subjects except Alzheimer's patients to prepare ³²P-labeled cDNA probes;
4. hybridizing the cDNA with the ³²P-labeled cDNA probes on the high-density human brain cDNA filters;
5. analyzing the results of the hybridization using an image analyzer and then determining the DNA sequence.

The polypeptides of the present invention (the polypeptide is referred to as "Alzheimer's disease-related polypeptide" hereinafter) are prepared by the following process;
1. digesting the Alzheimer's disease-related polypeptide-coding region of the Alzheimer's disease-related DNA fragment with restriction enzymes according to a routine method and then inserting the digested fragment into an expression vector;
2. introducing the expression vector in a host cell capable of expressing the objective Alzheimer's disease-related polypeptide according to a routine method to recover a transformant;
3. cultivating the transformant by a routine method to express and accumulate the objective Alzheimer's disease-related polypeptide;
4. purifying the accumulated Alzheimer's disease-related polypeptide by a routine method.

The antibodies of the present invention are prepared by the following process;
1. administering an antigen, namely a purified product of the Alzheimer's disease-related polypeptide of the whole length or a partial fragment thereof, subcutaneously, intravenously or peritoneally to rats, mice or hamsters aged 3 to 20 weeks, together with an appropriate adjuvant, for example complete Freund's adjuvant or aluminium hydroxide gel with B. pertussis vaccine to immunize the rats, mice or hamsters. The antigen should be administered five to 10 times every one to two weeks after the first administeration;
2. collecting serum from blood drawn from the ocular fundus plexus venosum on 3rd to 7th day after each administeration;
3. recognizing the reaction of the sera with the antigen by ELISA [see Enzyme-linked Immunoassay (ELISA), Igaku Shoin, 1976] and the like;
4. purifying the antibody from the sera, which was recognized to react with the antigen, according to the routine method.

The present invention is now described in detail hereinafter.

### 1. Preparation of DNAs

### (1) Preparation of a human brain cDNA library

From normal human brain is extracted the total RNA by routine methods such as acid guanidine phenol chloroform method (Analytical Biochemistry, 162, 156 - 159, 1987), guanidine cesium chloride method, guanidium thiocyanate method [Methods in Enzymology, 164, Academic Press (1987), Okayama et al.] and the like.

By column method or batch method using oligo-dT cellulose, poly(A)⁺ RNA is prepared from the RNA.

Using the poly(A)⁺ RNA as the template, the cDNA is synthesized by using a reverse transcriptase according to a method such as Okayama-Burg method [Mol. Cell. Biol., 2, 161 - 170 (1982)] and Gubler-Hoffman method [Gene 25, 263 - 269 (1983)].

According to a routine method such as the method by Sambrook [EMBO. J., 4, 91 -103 (1985)] and the method by Hyunh, T.V [DNA Cloning, A Practical Approach (D.M.Glover, ed.), 1, 49, IRL Press, Oxford], the cDNA is incorporated in a plasmid or a phage vector.

Any plasmid or phage vector autonomously replicable in a host cell to stably maintain the cDNA may be used to incorporate the cDNA therein. Specific examples thereof include plasmids of pBR322, pUC119, etc., phage vectors of λ gt10, λ ZAPII, etc., and the like.

When the cDNA is incorporated in the plasmids of pBR322 , pUC119, and the like, the plasmids are introduced into an appropriate host cell such as Escherichia coli and a microorganism belong to the genus Bacillus by electroporation method or calcium chloride method to transform the host cell; when the cDNA is incorporated in a phage vector, the phage vector is transduced in cultivated host cells by in vitro packaging and the like, to prepare a cDNA library.

### (2) Preparation of high-density human brain cDNA filters

Plasmid DNAs are prepared from the cDNA library prepared above in (1), which are then denatured in 0.2N NaOH. About 1 µg of the DNAs are dotted on filters, 8 x 12 cm in size (Hybond-N⁺ nylon membrane filter, manufactured by Amersham), by using an automatic gridding robot Biomek-1000 (manufactured by Beckman). Then, pBluescript plasmid DNA and the DNA encoding glycerol triphosphate dehydrogenase (referred to as "G3PD-DNA" hereinafter) are alternately dotted, adjacent to each sample dot.

The pBluescript plasmid DNA is dotted for background correction, while G3PD-DNA is dotted for the determination of internal standard. By such arrangement of the dots, the assay error due to the positions of the cDNA dots on the filters and the difference in hybridization level between the filters can be made the minimum. By dotting at a density as high as possible on a single filter, the procedures described below can be simplified. More specifically, a method comprising 300 to 400 dots on a single filter is mentioned.

After subjecting the filters to a heating process at 80 °C for one hour, the filters are irradiated with ultraviolet ray (at 1.2 x 10⁴ µJ/cm²) from a FUNA-UV-LINKER manufactured by Funakoshi to prepare high-density cDNA filters.

### (3) Preparation of mRNAs from the human brains of Alzheimer's patients and human subjects except Alzheimer's patients and preparation of ³²P- labeled cDNA probes

By the same procedures as described in the method described above in (1), the total RNAs are prepared from the human brain of patients with Alzheimer's disease and the human brain of human subjects except Alzheimer's patients. The RNAs are solubilized in diethyl pyrocarbonate-treated water to a final concentration of 2-20 µg/ml.

Using the RNAs as the template, ³²P-labeled cDNA probes are prepared according to a routine method. As the routine method includes for example a method by means of a kit (First Strand Synthesis Kit, manufactured by Stratagene).

### (4) Hybridization of the ³²P-labeled cDNA probes with the cDNA on the high-density cDNA filters

The high-density human brain cDNA filters, which are prepared according to the method described above in (2), are immersed in a hybridization solution with addition of the DNA from salmon sperm [6 x SSC solution (1 x SSC solution is composed of 150 mM sodium chloride and 15 mM sodium citrate), 10 x Denhardt's solution (1 x Denhardt's solution is composed of 0.02 % polyvinyl pyrrolidone, 0.02 % bovine serum albumin, and 0.02 % Ficoll), 1 % SDS and 100 µg/ml DNA from salmon sperm], for pre-hybridization treatment at 65 °C. After the pre-hybridization treatment, the ³²P-labeled cDNA probe is added to the resulting mixture, for hybridization treatment overnight at 65 °C. After the treatment, the filters are immersed in a solution containing 2 x SSC and 1 % SDS for heating at 65 °C for 15 minutes. After heating, the filters are immersed in a solution containing 0.1 x SSC and 0.1 % SDS for washing treatment at 65 °C for 15 minutes and subsequent drying in air.

### (5) Quantitative analysis of the results of the hybridization using an image analyzer and determination of the DNA sequence

The filters prepared according to the method described above in (4) are exposed to imaging plates for a Fuji bio-imaging analyzer of Model BAS 2000 System, to determine the radioactivity of each dot on the filters, by means of the Fuji bio-imaging analyzer of Model BAS 2000 System. The radioactivity of each dot is converted into relative luminous intensity score (referred to as "PSL") by using Auto Quant Quantitative Program. By subtracting from the PSL value of each cDNA clone thus recovered, the PSL value of the adjacent dot corresponding to the pBluescript plasmid DNA and subsequently normalizing the resulting PSL value using the PSL value of the internal standard G3PD-DNA positioned adjacently (reducing the experimental inter-filter and intra-filter errors due to the dot position), a data base is prepared.

Using the data base, the expression intensity of the probe from Alzheimer's patients is divided by the expression intensity of the probe from normal human subjects per each dot, to detect any probe with the variation of the expression intensity in the brain of Alzheimer's patients, compared with normal human brain.

In addition to such analysis method, the analysis method of the results of the hybridization includes any method capable of quantitatively analyzing the level of hybrids formed.

Among the plasmid DNAs from the human brain cDNA library dotted on the filters in the procedure (2), the DNA hybridized with the probe with the variation of the expression intensity in the brain of Alzheimer's patients compared with normal human brain can be identified.

The plasmid DNA contains a DNA with a complementary sequence to the RNA strand of the mRNA with the varied expression level in the brain of Alzheimer's patients, compared with normal brain.

The nucleotide sequence of the DNA in the plasmid DNA, having a complementary nucleotide sequence to the RNA strand of the mRNA with the varied expression level in the brain of Alzheimer's patients, compared with normal brain, can be determined by routine nucleotide sequencing methods, for example the dideoxy method by Sanger, et al. [Proc. Natl. Acad. Sci., USA, 74, 5463, 1977]. Sequencing of nucleotideis carried out by using a nucleotide auto-sequencer, for example ABI 373 DNA Sequencer or 373A DNA Sequencer, manufactured by Applied Biosystems.

Specific examples of the nucleotide sequence of the DNA with a complementary nucleotide sequence to the RNA strand of the mRNA with the increase in the expression level in the brains of patients with Alzheimer's disease include the nucleotide sequences of Sequence Nos. 1 to 15, while specific examples of the nucleotide sequence of the DNA with a complementary nucleotide sequence to the RNA strand of the mRNA with the decrease in the expression level include the nucleotide sequences of Sequence Nos. 16 and 17.

Escherichia coli DH5 α/pGCS55 containing the plasmid DNA with the sequence of Sequence No. 1, Escherichia coli DH5 α/pGCS99 containing the plasmid DNA with the sequence of Sequence No. 2, Escherichia coli DH5 α/pGCS198 containing the plasmid DNA with the sequence of Sequence No. 3, Escherichia coli DH5 α/pGCS328 containing the plasmid DNA with the sequence of Sequence No. 4, Escherichia coli DH5 α/pGCS335 containing the plasmid DNA with the sequence of Sequence No. 5, Escherichia coli DH5 α/pGCS547 containing the plasmid DNA with the sequence of Sequence No. 6, Escherichia coli DH5 α/pGCS998 containing the plasmid DNA with the sequence of Sequence No.7, Escherichia coli DH5 α/pGCS1148 containing the plasmid DNA with the sequence of Sequence No. 8, Escherichia coli DH5 α/pGCS1180 containing the plasmid DNA with the sequence of Sequence No. 9, Escherichia coli DH5 α/pGCS1243 containing the plasmid DNA with the sequence of Sequence No. 10, Escherichia coli DH5 α/pGCS2232 containing the plasmid DNA with the sequence of Sequence No.11, Escherichia coli DH5 α/pGCS3282 containing the plasmid DNA with the sequence of Sequence No.12, Escherichia coli DH5 α/pGCS11037 containing the plasmid DNA with the sequence of Sequence No.13, Escherichia coli DH5 α/pGCS1984 containing the plasmid DNA with the sequence of Sequence No.16 and Escherichia coli DH5 α/pGCS2593 containing the plasmid DNA with the sequence of Sequence No.17, have been deposited with the National Institute of Bioscience and Human-Technology of the Agency of Industrial Science and Technology at 1-3, Higashi 1-chome, Tsukuba-shi, Ibaraki-ken, 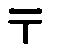 305, Japan under FERM BP-5304 through 5316, 5318 and 5319, respectively, on the date of 28 November, 1995, in terms of the Budapest Treaty.

### 2. Preparation of a Alzheimer's disease-related polypeptide

So as to express the Alzheimer's disease-related DNA thus recovered above in a host cell, a DNA fragment containing the Alzheimer's disease-related DNA is digested with restriction enzymes or DNases into a DNA fragment of an appropriate length, containing the DNA encoding the Alzheimer's disease-related polypeptide (referred to as "Alzheimer's disease-related gene" hereinafter), which is then inserted in the downstream of a promoter in an expression vector. Subsequently, the expression vector inserted with the DNA is introduced in a host cell appropriate for the expression vector. As such host cell, use is made of any host cell capable of expressing the objective gene can be used. As examples of the host cell, prokaryotes belonging to the Escherichia, Serratia, Corynebacterium, Brevibacterium, Pseudomonas, Bacillus or the like, and yeast strains belong to the geuns Kluyveromyces, Saccharomyces, Schizosaccharomyces, Trichosporon, Schwanniomyces or the like; animal cell hosts and insect cell hosts and the like can be mentioned.

Any expression vector, which ca be autonomously replicable in the above-mentioned host cell or is capable of being incorporated into a chromosome and which contains a promoter at a position where the Alzheimer's disease-related gene can be transcribed, can be used.

When a prokaryotic organism such as bacteria is used as such host cell, preferably, the expression vector for the Alzheimer's disease-related gene should be autonomously replicable in the prokaryotic organism and be composed of a promoter, a ribosome binding sequence, the Alzheimer's disease-related gene, and a transcription termination sequence. A regulatory gene of the promoter may be contained therein.

As example of the expression vector, mentioned are pBTrp2, pBTacl, pBTac2 (all are commercially available from Boehringer Mannheim), pKYP10 (Japanese Published Unexamined Patent Application No. 110600/83), pKYP200[Agric. Biol. Chem., 48, 669(1984)], pLSA1 [Agric. Biol. Chem., 53, 277(1989)], pGEL1 [Proc. Natl. Acad. Sci., USA, 82, 4306 (1985)], pBluescript (Stratagene), pTrs30 [prepared from Escherichia coli JM109/pTrS30 (FERM BP-5407)] and pTrs32 [prepared from Escherichia coli JM109/pTrS32 (FERM BP-5408)].

As the promoter, usable is any promoter capable of being expresed in a host cell such as Escherichia coli. For example, mentioned are promoters derived from Escherichia coli, phages, etc., such as trp promoter (Ptrp), lac promoter (Plac), P_{L} promoter, and P_{R} promoter. Also, usable are artificially designed and modified promoter, such as a promoter (Ptrp x 2) composed of two Ptrp's in tandem series, tac promoter and the like.

As ribosome binding sequence, any ribosome binding sequence capable of being expressed in a host cell such as Escherichia coli is used. Preferably, a plasmid with an appropriate distance (for example 6 to 18 nucleotides) between the ribosome binding sequence and the initiation codon may be used.

The transcription termination sequence is not always necessary required for the expression of the gene, but, it is desirable that a transcription termination sequence is arranged immediately downstream the structural gene.

As the host cell, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No.49, Escherichia coli W3110, Escherichia coli NY49, Bacillus subtilis, Bacillus amyloliquefacines, Brevibacterium immariophilum ATCC14068, Brevibacterium saccharolyticum ATCC14066, Brevibacterium flavum ATCC14067, Brevibacterium lactofermentum ATCC 13869, Corynebacterium glutamicum ATCC13032, Corynebacterium acetoacidophilum ATCC 13870, Microbacterium ammoniaphium ATCC15354 and the like can be mentioned.

When a yeast strain is used as a host cell, for example, YEp13 (ATCC37115), YEp24 (ATCC37051), YCp50 (ATCC37419), and the like can be used as the expression vector.

As the promoter, any promoter capable of being active in a yeast bacterial strain may be used. AS examples of promoters, promoters of glycolytic genes such as hexose kinase, gal 1 promoter, gal 10 promoter, heat shock protein promoter, MF α1 promoter, CUP 1 promoter and the like can be used.

As examples of the host cell, Saccharomyces cerevisae, Schizosaccharomyces pombe, Kluyveromyces lactis, Trichosporon pullulans, Schwanniomyces alluvius and the like can be mentioned.

When an animal cell is used as the host cell, for example, pcDNAI/Amp, pcDNAI, pcDM8 (all commercially available from Funakoshi), pAGE107 [Cytotechnology, 3, 133(1990)], pAGE103 [Journal of Biochemistry, 101, 1307(1987)] and the like can be used as the expression vector.

As the promoter, any promoter capable of being active in animal cells may be used, For example, the promoter for the IE (immediate early) gene of human CMV and the like can be used. Additionally, the enhancer for the IE gene of human CMV may be used together with the promoter.

As examples of the host cell, Namalwa cell, HBT5637(Japanese Published Unexamined Patent Application No. 299/88), COS cell, CHO cell and the like can be used.

As the method for introducing the DNA into animal cells, any and ever method capable of introducing DNA into animal cells may be used. For example, employable are electroporation method [Miyaji et al., Cytotechnology, 3, 133(1990)], calcium phosphate method (Japanese Published Unexamined Patent Application No. 227075/90), lipofection method [Philip L. Felgner, et al., Proc. Natl. Acad. Sci., USA, 84, 7413(1987)] and the like. The resulting transformants can be obtained and cultivated according to the method described in Japanese Published Unexamined Patent Application No. 227075/90 or 257891/90.

For insect cells, insect cells such as Sf9 and Sf21 (all manufactured by Farmingen), which are infected with the recombinant Baculovirus [Bio/Technology, 6, 47(1988)] prepared by using the BaculoGold Starter Kit manufactured by Farmingen, can be used.

By cultivating a transformant carrying the recombinant DNA comprising the Alzheimer's disease-related gene, according to a routine cultivation method, to generate and accumulate the Alzheimer's disease-related polypeptide and recovering the Alzheimer's disease-related polypeptide from the culture, the Alzheimer's disease-related polypeptide can be produced.

When the transformant for producing the Alzheimer's disease-related polypeptide is a prokaryote such as Escherichia coli or an eucaryote such as yeast, the medium for cultivating these organisms may be any medium, natural or synthetic, as long as the medium contains a carbon source, a nitrogen source, inorganic salts and the like, all of which the organisms can assimilate to effectively cultivate the transformant.

Any carbon source which the individual microorganisms can assimilate may be used satisfactorily, such as carbohydrates such as glucose, fructose, sucrose, molasses containing these carbon sources, starch and starch hydrolysate; organic acids such as acetic acid and propionic acid; and alcohols such as ethanol and propanol.

As the nitrogen source, ammonia; ammonium salts of various inorganic acids and organic acids, such as ammonium chloride, ammonium sulfate, ammonium acetate, and ammonium phosphate; other nitrogen-containing compounds; peptone, meat extract, yeast extract, corn steep liquor, casein hydrolysate, soy bean bran and soy bean bran hydrolysate, various cultivated cells, digested products thereof and the like may be used.

As inorganic subatances, potassium dihydrogen phosphate, dipotassium hydrogen phosphate, magnesium phosphate, magnesium sulfate, sodium chloride, ferrous sulfate, manganese sulfate, copper sulfate, calcium carbonate and the like may be used.

For cultivation of fungi, cultivation medium, containing a carbonsource such as wheat bran, rice bran, or the like, a nitrogen source and an inorganic source and being supplemented with appropriate salts, may be used.

Cultivation is carried out under aerobic conditions such as shaking culture or submerged-aerial stirring culture. The cultivation temperature is preferably 15 to 40 °C and the cultivation period is generally 16 to 96 hours. The pH should be maintained at 3.0 to 9.0 during the cultivation. The pH is adjusted by using inorganic or organic acids, alkali solutions, urea, calcium carbonate, ammonia and the like.

During cultivation, an antibiotic such as ampicillin and tetracycline may satisfactorily be added to a cultivation medium, if necessary.

For cultivating a microorganism transformed with an expression vector using an inducible promoter as the promoter, an inducer is satisfactorily added to the cultivation medium if necessary. For cultivating a microorganism transformed with an expression vector using lac promoter, for example, isopropyl-β-D-thiogalactopyranoside (IPTG) is added to the cultivation medium; for cultivating a microorganism transformed with an expression vector using trp promoter, indole acrylic acid (IAA) is satisfactorily added to the cultivation medium.

When the transformant for producing the Alzheimer's disease-related polypeptide is an animal cell, RPMI 1640 medium and Eagle's MEM medium which are general used or these media containing a fetal bovine serum or the like can be used, as the medium for cultivating the cell.

Cultivation is carried out under conditions in the presence of 5 % CO₂. The cultivation temperature is preferably 35 to 37 °C, and the cultivation period is generally 3 to 7 days.

During cultivation, if necessary, an antibiotic such as kanamycin and penicillin is added to the cultivation medium.

So as to isolate and purify the Alzheimer's disease-related polypeptide from the culture of the transformant for producing the Alzheimer's disease-related polypeptide, general methods for isolating and purifying enzymes may be used.

When the Alzheimer's disease-related polypeptide is accumulated in the cells of the transformant for producing the Alzheimer's disease-related polypeptide, the culture is centrifuged to collect the cells in the culture, and the cells are washed and then disrupted by means of an ultrasonicator, a French press, a Manton-Gaulin homogenizer, a dinomill and the like, whereby a cell-free extract can be recovered. From the supernatant of the centrifuged cell-free extract, thereafter, a purified product can be recovered by salting out with ammonium sulfate or the like, desalting, precipitation by an organic solvent, anion exchange chromatography using resins such as diethylaminoethyl (DEAE)-Sepharose and DIAION HPA-75 (manufactured by Mitsubishi Chemical), cation exchange chromatography using resins such as S-Sepharose FF (manufactured by Pharmacia), hydrophobic chromatography using resins such as butyl Sepharose and phenyl Sepharose, gel filtration using molecular sieve, chromato-focusing and electrophoresis such as isoelectric focusing.

When the Alzheimer's disease-related polypeptide is secreted outside cells, the culture is treated with a procedure such as centrifugation, to recover a soluble fraction. From the soluble fraction, the purified product of the Alzheimer's disease-related polypeptide can be recovered by the same method as the above-mentioned method for isolation and purification from the supernatant of the cell-free extract.

### 3. Preparation of Alzheimer's disease-related antibody

The a purified product of whole length or a partial fragment of the Alzheimer's disease-related polypeptide (antigen), as recovered above in 2. Is administered at about 50 to 100 µg/animal, subcutaneously, intravenously or peritoneally, to rats, mice or hamsters aged 3 to 20 weeks, together with an appropriate adjuvant, for example complete Freund's adjuvant or aluminium hydroxide gel with B. pertussis vaccine.

The antigen need be administered five to 10 times every one to two weeks after the first administration. After each administration, serum is collected from blood drawn from the ocular fundus plexus venosum on the 3ed to 7th day. The reaction of the sera with the antigen is recognized by ELISA [see Enzyme-linked Immunoassay (ELISA), Igaku Shoin, 1976; Antibodies - A Laboratory Manual, Cold Spring Harbor Laboratory, 1988] and the like.

From the mice, rats or hamsters of which the sera have sufficient antibody titers to the antigen used for their immunization, the sera are collected to recover a purified antibody from the sera by using routine methods such as salting out by means of 40 to 50 % saturated ammonium sulfate, caprylic acid precipitation, chromatography methods using for example DEAE-Sepharose column, protein A column or gel filtration column.

### 4. Diagnosis and therapeutic treatment of patients with Alzheimer's disease

(1) Using as a diagnostic agent the DNA which is identified according to the method described in 1.- (5) and has a complementary nucleotide sequence to the RNA strand of the mRNA with the increase in the expression level in the brains of patients with Alzheimer's disease (referred to as "diagnostic DNA" hereinafter), patients with Alzheimer's disease can be identified.
   According to the method described in 1.-(2), more specifically, the diagnostic DNA is dotted on filters to prepare DNA filters. According to the method described in 1.-(3), ³²P-labeled cDNA probes are individually prepared from the brains of a test patient and normal subjects. The ³²P-labeled cDNA probes are used for hybridization with the diagnostic DNA on the DNA filters according to the method described in 1.-(4), to compare the expression level of the gene corresponding to the hybridizing probes between the test patients and normal subjects according to the method described in 1.-(5). Definite diagnosis of as to whether or not the test patient is afflicted with Alzheimer's disease depends on whether or not the pattern of the expression level of the gene corresponding to the diagnostic DNA in the test patient agrees with the pattern of the expression of the gene in patients with Alzheimer's disease.
(2) Using the antibody recovered by the method described in 3., the amount of the Alzheimer's disease-related polypeptide in the tissue extract of the test patient is assayed by radioimmunoassay after labeling the Alzheimer's disease-related polypeptide with a radioisotope.
   Whether or not the test patient afflicted with Alzheimer's disease can be diagnosed, depending on, according to the determination method as mentioned above, whether or not the amount of the Alzheimer's disease-related polypeptide matches with the pattern of Alzheimer's patients.
(3) The polypeptide encoded by the DNA which expression level is decreased in patients with Alzheimer's disease can be used as a therapeutic agent for treating Alzheimer's patients for a therapeutic method comprising supplementing the patients with the polypeptide, while the polypeptide encoded by the DNA which expression level is increased in patients with Alzheimer's disease is useful for the preparation of an antibody recognizing the polypeptide. Therefore, the antibody can be used as a therapeutic agent for treating Alzheimer's patients for a therapeutic method comprising suppressing the action of the polypeptide.
(4) The DNAs of the present invention can be used as a gene for gene therapy of Alzheimer's disease, while the antisense nucleic acid corresponding to the present cDNA can be used as a nucleic acid for gene therapy of Alzheimer's disease.
(5) The DNAs, polypeptides and antibodies of the present invention can be used as a reagent for research works regarding the mechanism of the onset of Alzheimer's disease.

### BEST MODES OF CARRYING OUT THE INVENTION

The present invention is now described with reference to the following examples.

### Example 1

### (1) Preparation of a human brain cDNA library

From the normal region of the frontal cortex of a Japanese male patient (aged 59 years) with glioblastoma, the total RNA was extracted by acid guanidine phenol chloroform method (Analytical Biochemistry, 162, 156 - 159, 1987). Poly A⁺ RNA was purified from the total RNA by using a commercially available "oligo-dT". Using the polyA⁺ RNA of 5 µg as the template, together with a reverse transcriptase Superscript™ and Ribonuclease H manufactured by BRL and Escherichia coli DNA polymerase I and T4 DNA polymerase manufactured by Takara Shuzo, a cDNA library (λ phage) was prepared by means of a λ ZAP II vector kit manufactured by Stratagene. The thus recovered phage particles of 1 x 10⁵ or more were mixed with 200 µl of a culture of Escherichia coli XL1-Blue (OD₆₀₀ = 1) and 1 µl of helper phage R408, for cultivation at 37 °C for 15 minutes, followed by addition of 2 x YT cultivation medium (5 ml) (16 g/l Bacto-tryptone, 10 g/l yeast extract, 5 g/l NaCl, pH 7.0) for cultivation at 37 °C for 3 hours. After cultivation, the resulting culture was heated at 70 °C for 20 minutes and centrifuged at 4000 x g for 5 minutes, to recover a supernatant containing the recombinant phagemid.

The phagemid solution (200 µl) was mixed with an equal volume of the Escherichia coli XL1-Blue culture, for cultivation at 37 °C for 15 minutes, and a part of the mixture was then spread on a petri dish containing a 50 µg/ml ampicillin-containing LB medium (10 g/l Bacto-peptone, 5 g/l yeast extract, 10 g/l NaCl, pH 7.0) to a final occurrence of several hundreds of colonies, and followed by cultivation at 37 °C overnight. Each of the colonies appearing on the next morning was independently cultivated in a 50 µg/ml ampicillin-containing 2 x YT cultivation medium (5 ml) at 37 °C for 12 hours. From the cultivated cells recovered from the culture, plasmid DNA was purified by using an automatic extraction system PI-100 manufactured by Kurabo.

Digesting 100 plasmid DNAs with restriction enzymes EcoRI and XhoI for analysis by agarose gel electrophoresis, almost all the plasmids contained inserts of 0.4 kb or more. By subsequently using Taq Dye Primer™ Cycle Sequencing Kit (Applied Biosystems), cycle sequencing was started from the 3' terminus of the cDNA, to determine a partial nucleotide sequence by ABI 373 DNA sequencer (Perkin Elmer). The homology of the resulting DNA sequences was examined on the basis of the GenBank DNA data base. Consequently, it was demonstrated that the sequences of the genes relating to mitochondria occupied about 30 % while about 70 % of the remaining sequences were novel genes.

### (2) Preparation of high-density cDNA filters

By the method described in Example 1, from the human cerebral cortex cDNA library, 15,256 plasmid DNAs were independently extracted. From the cDNA library, clones containing the mitochondria-related genes were removed. About 1 µg of the plasmid DNAs from 8353 clones were denatured with 0.2 N NaOH, which were then dotted on a Hybond-N⁺ nylon membrane filters (Amersham), 8 x 12 cm in size, by using an automatic gridding robot Biomek-1000 manufactured by Beckman. Furthermore, pBluescript vector DNA and G3PD-DNA were alternately dotted, adjacent to each sample dot.

The filters were treated under heating at 80 °C for one hour, followed by ultraviolet irradiation (at 1.2 x 10⁴ µJ/cm²) from FUNA-UV-LINKER manufactured by Funakoshi to prepare high-density cDNA filters.

### (3) Preparation of ³²P-labeled cDNA probe

Using ISOGEN manufactured by Nippon Gene, cell-free extracts were prepared individually from the brains (each about 1 g) of patients with Alzheimer's disease and normal subjects, by means of a homogenizer of POLYTORON PT10 20 3500, to prepare individually the total RNAs according to the method instructed in the ISOGEN. To a final concentration of 10 µg/2.3 ml, each of the total RNAs was solubilized in diethyl pyrocarbonate-treated water.

Using 10 µg of each of the total RNAs thus recovered as a template, ³²P-labelled cDNA probe was prepared as follows, using a kit manufactured by Stratagene (First Strand Synthesis Kit).

25 µL of α-³²P-dCTP (6000 Ci/mmol; manufactured by New England Nuclear) and 1 µl each of 1 mM dATP, dTTP, and dGTP were mixed with 0.2 µl of 0.5 µg/µl Oligo dT, and the resulting mixture was subsequently dried up by using a vacuum pump. The dried product was solubilized in 2.3 µl of the RNA solution (10 µg/2.3 µl) from human brain recovered in Example 1 (1), followed by addition of 0.8 µl of a 5 x buffer solution and subsequent thorough mixing. The mixture solution was heated at 70 °C for 10 minutes, and was then immersed in ice for rapid cooling.

To the solution was added 4 µl of 0.1M DTT for heating at 42 °C for 2 minutes, followed by addition of 0.5 µl of Super Script II, and then a reaction was carried out at 42 °C for 30 minutes. To the resulting reaction solution were added 0.4 µl of 25mM dNTP, 1 µl of 5 x buffer solution, 0.5 µl of 0.1M DTT, 2.6 µl of diethyl pyrocarbonate-treated water, and 0.5 µl of Super Script II, for reaction at 42 °C for 30 minutes. To the resulting reaction solution was added 3 µl of 0.5M EDTA, followed by subsequent addition of 10 µl of TE (10 mM Tris-HCl, pH 7.0, 1mM EDTA), to remove free ³²P-dCTP by spin column method using Quick Spin™ Columns Sephadex G50, fine, manufactured by Boehringer Mannheim.

### (4) Hybridization of the ³²P-labeled cDNA probe with the cDNAs on the high-density cDNA filters

The high-density cDNA filters prepared in Example 1 (2) were immersed in a hybridization solution with addition of the DNA derived from salmon sperm, for pre-hybridization treatment at 65 °C. The ³²P-labeled human brain cDNA prepared in Example 1 (3) was added to the resulting treated solution, and the following hybridization treatment was carried out.

A distilled water was added to the ³²P-labeled human brain cDNA prepared in Example 1 (3) to a final volume of 500 µl, followed by addition of 17 µl of 10N NaOH for denaturing of the cDNA. After adding 17 µl of 10N HCl to the resulting mixture for neutralization of the mixture, the mixture was added to the solution treated for pre-hybridization, and hybridization treatment was conducted overnight at 65 °C. The filters thus treated were washed in a solution containing 2 x SSC and 1 % SDS at 65 °C for 15 minutes and in a solution containing 0.1 x SSC and 1 % SDS at 65 °C for 15 minutes, followed by drying in air.

### (5) Quantitative analysis of the hybridization results using an image analyzer

The filters prepared in Example 1 (4) were exposed to an imaging plates for Fuji bio-imaging analyzer BAS2000 System for 5 to 10 hours, and then, the radioactivity from each dot on the filters was determined by using the Fuji bio-imaging analyzer BAS 2000 System.

The radioactivity of each dot was converted into relative luminous intensity score (referred to as "PSL",) by using the Auto Quant Quantitative Program. By subtracting, from the PSL value of the plasmid DNA derived from each cDNA clone thus recovered, the PSL value of the adjacent dot corresponding to the pBluescript plasmid DNA and subsequently normalizing the resulting PSL value using the PSL value of the internal standard G3PD-DNA positioned adjacently, a data base was prepared.

By dividing the expression intensity of the probe from Alzheimer's patients by the expression intensity of the probe from normal human subjects per each dot using the data base, detecting a probe with the modification in the expression intensity in the brain of Alzheimer's patients, compared with the brain of normal human subjects, a plasmid DNA hybridized with the detected probe was identified from the human brain cDNA library used for dotting in Example 1 (2).

The plasmid DNA contains a DNA with a complementary sequence to the RNA strand of the mRNA with the varied expression level in the brain of Alzheimer's patients, compared with normal brain.

Among the plasmid DNAs, 15 types of plasmid DNAs individually containing a DNA with a complementary nucleotide sequence to the RNA strand of the mRNA with the increase in the expression level by two-fold or more in the brain of Alzheimer's patients, and 2 types of plasmid DNAs individually containing such DNA with the decrease in the expression level of the mRNA by 0.5-fold or less were identified.

The results are shown in Table 1.

**Table 1**

| Expression level in Alzheimer's patients, compared with normal subjects | | |
|---|---|---|
| Plasmid | Sequence No. | Ratio of expression levels (patients/normal subjects) |
| pGCS55 | 1 | 10.7 |
| pGCS99 | 2 | 4.7 |
| pGCS198 | 3 | 11.5 |
| pGCS328 | 4 | 10.6 |
| pGCS335 | 5 | 14.9 |
| pGCS547 | 6 | 15.2 |
| pGCS998 | 7 | 22.7 |
| pGCS1148 | 8 | 37.7 |
| pGCS1180 | 9 | 10.8 |
| pGCS1243 | 10 | 22.1 |
| pGCS2232 | 11 | 7.2 |
| pGCS3282 | 12 | 20.3 |
| pGCS11037 | 13 | 27.7 |
| pGCS427 | 14 | 14.5 |
| pGCS981 | 15 | 17.5 |
| pGCS1984 | 16 | 0.14 |
| pGCS2593 | 17 | 0.067 |

The DNA sequences with a complementary nucleotide sequence to the RNA strand of the mRNA with the increase in the expression level in the brain of Alzheimer's patients, which are contained in plasmid DNAs, pGCS55, pGCS99, pGCS198, pGCS328, pGCS335, pGCS547, pGCS998, pGCS1148, pGCS1180, pGCS1243, pGCS2232, pGCS3282, pGCS11037, pGCS427 and pGCS981 respectively, are shown as Sequence Nos.1 to 15. The DNA sequences with a complementary nucleotide sequence to the RNA strand of the mRNA with the decrease in the expression level in the brain of Alzheimer's patients, which are contained in plasmid DNAs, pGCS1984 and pGCS2593 respectively, are shown as Sequence Nos.16 and 17. The DNA sequences were determined by the method by Sanger, et al.

### Example 2

### Diagnosis of Alzheimer's patients

Using 17 types of diagnostic DNAs as specified in Example 1 (5), specifically the plasmid DNAs, namely pGCS55, pGCS99, pGCS198, pGCS328, pGCS335, pGCS547, pGCS998, pGCS1148, pGCS1180, pGCS1243, pGCS2232, pGCS3282, pGCS11037, pGCS427 and pGCS981, each containing a DNA with a complementary nucleotide sequence to the RNA strand of the mRNA with the increase in the expression level in the brain of Alzheimer's patients, as shown as Sequence Nos.1, 2, 3, 4, 5, 6, 7, 8, 9, 10, 11, 12, 13, 14 and 15, respectively and the plasmid DNAs, namely pGCS1984 and pGCS2593, each containing a DNA with a complementary nucleotide sequence to the RNA strand of the mRNA with the decrease in the expression level in the brain of Alzheimer's patients, as shown as Sequence Nos.16 and 17 respectively, diagnosis of Alzheimer's disease was made.

According to the method described in Example (2), the diagnostic DNAs were dotted on the filters, to prepare DNA filters. According to the method described in Example 1 (3), the brains of three Alzheimer patients and two normal subjects were used to prepare individual ³²P-labeled cDNA probes.

The ³²P-labeled cDNA probes were used for hybridization with the diagnostic DNAs on the DNA filters according to the method described in Example 1(4), and according to the method described in Example 1(5), and then the expression level of each of the genes individually corresponding to the hybridizing prove was compared between Alzheimer patients and normal subjects.

The results are shown in Table 2.

**Table 2**

| Ratio of expression levels in Alzheimer patients compared with normal subjects | | | |
|---|---|---|---|
| Sequence No. | Ratio of expression levels (patients/normal subjects) | | |
| | Patient 1 | Patient 2 | Patient 3 |
| 1 | 5.2 | 4.5 | 5.9 |
| 2 | 2.0 | 2.6 | 2.5 |
| 3 | 2.9 | 5.2 | 3.4 |
| 4 | 6.3 | 5.5 | 6.6 |
| 5 | 2.4 | 2.8 | 6.4 |
| 6 | 5.4 | 5.7 | 4.8 |
| 7 | 6.0 | 10.3 | 16.5 |
| 8 | 7.7 | 9.2 | 14.1 |
| 9 | 8.3 | 6.5 | 10.2 |
| 10 | 3.3 | 8.3 | 18.3 |
| 11 | 4.9 | 4.7 | 7.7 |
| 12 | 6.0 | 7.4 | 15.3 |
| 13 | 3.2 | 7.3 | 23.8 |
| 14 | 2.4 | 3.9 | 8.2 |
| 15 | 3.3 | 6.4 | 12.3 |
| 16 | 0.14 | 0.25 | 0.35 |
| 17 | 0.25 | 0.38 | 0.25 |

As shown in Table 2, the expression levels of the genes corresponding to the Sequence Nos.1 to 17 in the Alzheimer patients are so varied to be discriminated from the levels thereof in normal subjects. It is indicated that the method is useful for diagnosing Alzheimer's disease.

### INDUSTRIAL APPLICABILITY

The DNAs of the present invention can be used for the diagnosis, therapeutic treatment and research works of patients with Alzheimer's disease and for the production of the polypeptides encoded by the DNAs. The polypeptides of the present invention can be used for the diagnosis, therapeutic treatment and research works of patients with Alzheimer's disease and for the production of an antibodies recognizing the polypeptides. The antibodies of the present invention can be used for the diagnosis and therapeutic treatment of patients with Alzheimer's disease and as a research source of patients with Alzheimer's disease.

SEQUENCE LISTING

SEQ ID NO:1
LENGTH:2688
TYPE: nucleic acid
STRANDEDNESS: double
TOPOLOGY: linear
MOLECULE TYPE:cDNA to mRNA
ORIGINAL SOURCE:
  ORGANISM:human
  CLONE:55
FEATURE:
  KEY: CDS
  LOCATION: 358.. 486
  LOCATION: 560.. 799
  LOCATION: 1042.. 1182
  LOCATION: 2105.. 2269
  LOCATION: 2370.. 2462
  IDENTIFICATION METHOD: E
SEQUENCE
GAATTCGGCA CGAGAATGAG CCCCACAGAA TAAAGGTTTT TCCTCTTGCT GTGGCCTACT 60
GGAAGCCTTC CTAACTCTAT AGCTGGTAAA GTAGAGCAGA AAGGGCCAGA ATGGCTTAAG 120
ATGGAGCTAA ATCCCTATAG CATTCCATTT TCTGCTCTTG CCTCTTGAAC TTAGAAGGCT 180
TGCCCATATA TCCTAACAAT CTGTTCTCTG AGCTGTTAGG CCAATCCCTG GTCTCAAACT 240
CCTGACCTCA GGTGATCCAC CCACTATGGC CTCCCAAGGT GCTGGGATTG CAGATGTGAG 300
CCACCGTGCC TGGCCAGAAA ATCTGGATTC TTATTCCTAG TTCTTCATTT CTGTCAC AT 359
Met
1
G CAC TTA GTT GAC ATT ACA TCT ACA TAT ATT AGC TTT TTC CTA CAT GAG 408
His Leu Val Asp Ile Thr Ser Thr Tyr Ile Ser Phe Phe Leu His Glu
            5                   10                  15
CCA TCT ATT TAC TTA GTA ACC AGT GTT CTT AAT GAA GTA TTT AGT CTT 456
Pro Ser Ile Tyr Leu Val Thr Ser Val Leu Asn Glu Val Phe Ser Leu
        20                  25                  30
GGG TTT CTT GTA AAA TTT CTC TGC ATT CCT TAGACAGTGT ACTATACATG 506
Gly Phe Leu Val Lys Phe Leu Cys Ile Pro
    35                  40
AAATATTCTT GTTGACCTAG TAATTTATAT TATTCCATTT AATTCTTAAA CCT ATG 562
Met
1
GCC TTT TTA TTG AGC ACA CTC TTA AAT CAT TAT TTG GCT TGT AAA CAT 610

Ala Phe Leu Leu Ser Thr Leu Leu Asn His Tyr Leu Ala Cys Lys His
5 10 15
TCA TCT GAA TTG TGG CTA CAA TCC TCT TTA AAT AAT CTA GGA AAA AAG 658
Ser Ser Glu Leu Trp Leu Gln Ser Ser Leu Asn Asn Leu Gly Lys Lys
20 25 30
AAA GAT AAA GCT TAC ATT TTC ACA GTT TTG GCT CTT AAA CAC ATT CCA 706
Lys Asp Lys Ala Tyr Ile Phe Thr Val Leu Ala Leu Lys His Ile Pro
35 40 45
CAA ATG CCA TTA AGA ATT TAT TTT GTT TTA GGC CAG TCA TGG TGG CTC 754
Gln Met Pro Leu Arg Ile Tyr Phe Val Leu Gly Gln Ser Trp Trp Leu
50 55 60 65
ATG CCT GTA ATC CCA GCA ATT TGG GAG GCT GAG GCA AGA ACT GCT 799
Met Pro Val Ile Pro Ala Ile Trp Glu Ala Glu Ala Arg Thr Ala
70 75 80
TGAGCCCAGG AGTTTGAGAC TAGCCTGGGC AACATAGCAA GACCCTGTCT CTACCAAAAA 859
AAAAAAAGTT TATTTTGTTT TAGAGTCATT TAATGTGTTT TTATGCACAA TAATAGTGGG 919
AGGTTGTTTT GTTGCATTTG TTTGTTTGTT TTGTTTTGTT TTGTTTTGCT TTCCATGTGG 979
GAAAAGTTAA CATTGGAACT GTTTCTAGTA AAAGATTTTT TTCAGGCTGG GCACGGTGGC 1039
TC ATG CCT GTG ATC CCA ACA CTT TGG GAG ACC GAG GGA GCT GGA TCA 1086
Met Pro Val Ile Pro Thr Leu Trp Glu Thr Glu Gly Ala Gly Ser
1 5 10 15
CCT GAG GTC AGG AGT TCG AGA CCA GCC TGG CCA ACA TGG AAA AAC TCC 1134
Pro Glu Val Arg Ser Ser Arg Pro Ala Trp Pro Thr Trp Lys Asn Ser
20 25 30
ATC TCC ACT AGA AAT ACA AAA GTA GCC TGG TGT GGT GGC ACA TGC CTG 1182
Ile Ser Thr Arg Asn Thr Lys Val Ala Trp Cys Gly Gly Thr Cys Leu
35 40 45
TAATCCCAGC TACTTGGGAA GCTGAGGCAG GAGAATCACT TGAACCTGGG AGGTGGAGGT 1242
TGCAGTGAGC CAAGATCACG CCATTGCACT CCAGCCTGGG CAACAAGAGT GAAACTCCGT 1302
CTCAAAAAAA AAAAAAAAAA AGATGTTTTT CATTTTTTTC ATGTTATCTA TCCAAGCACT 1362
GTTCCATGGT CAGCAAGTCA TATTTCATAA TGTGGATTTT CCAAAATAAT TATTGAATAC 1422
AGCTATTCTA TGGCTACTTT TAGTGTTTTT GTGGTATGTG GTGTGGGAGT GTTTATGGAA 1482
TTACCAGTAT CTTAAATTTT CAAAGGAACC TTGGAAGTCT ATCACTCTAA ATGAAAGTCT 1542
GTCACTCTAC ATGAATTATG TGCTCAAATT TGACCAACTC AGTTTAAGAC ACAAAACAGT 1602
AATTTGAAGA AGGAAAAATG AAGAGAGTTT CTAGTTTAAT GGGTTAAATT TTTGTTGTTG 1662

CAATAGTAAG TTTAGTCTTC TTATAATATT TCTAAATGAA AAATCATAGG TATTTGTTAC 1722
CATGTGTGAA GATTACTTTG TTAAAAGCAA AAGTGGTCGT GTGATATGCT AAATGTTAAT 1782
TACTGATTTT ATATGTTTAA ATCACGCCAA ACAAATTATG TCTGTGCCAT CCAGGGTCTG 1842
TTGTTAATCT TTTTCTGAGT ACTTGGATTG GGATAAAGGG CTTGTACTAT GCACTTTTTA 1902
TTAATGAATA AATAGAAAAC GTTAGTAACA CTTTGTGTTT TCTGTTTGGC TTTTGTGGGA 1962
AGAGAAGCAA GCATCTTTTG CCTAGTAGAT GTTAACATTG TGTATTAAAC AGTTTCTTTG 2022
TAAGACCTAA CTAAAGACAT TCCTAAGAGA GAACTTAAGT TTTAAGAAAA GTTGTGAGAA 2082
AAATATTCAT AAAATATGCA GT ATG GGG CCA GTA TTC AGA AGT AGA GTT TCT 2134
Met Gly Pro Val Phe Arg Ser Arg Val Ser
1 5 10
CTC TTG GAA ATT AGA TTG CAG TCA CAT TTC TTT GGT TAT TTT CTT TCT 2182
Leu Leu Glu Ile Arg Leu Gln Ser His Phe Phe Gly Tyr Phe Leu Ser
15 20 25
CTC TCT CTT CCT AGA ATA CCA GGT AAA ATA GAC ATT TAC TTT GAG GGA 2230
Leu Ser Leu Pro Arg Ile Pro Gly Lys Ile Asp Ile Tyr Phe Glu Gly
30 35 40
CTA ATA TCC AAT AGT GTT AAT TAT TTA AGC AGA TAT ATC TAGCTCAAAA 2279
Leu Ile Ser Asn Ser Val Asn Tyr Leu Ser Arg Tyr Ile
45 50 55
GGAACCAGAC ATGTCACTGT ACCAAAAACA CACAAAAGTG AAAATTTGCT TCTGTTCTGT 2339
GACCTAGAAT GTGTCCAGAC ATTAAAGATC ATG AAT ACT CAT ATA CAT GTA AAA 2393
Met Asn Thr His Ile His Val Lys
1 5
ATA GTA ACA CTG CTC TGG CAT GAT TTT GAC GTC AGT GTT TAT GTA ATA 2441
Ile Val Thr Leu Leu Trp His Asp Phe Asp Val Ser Val Tyr Val Ile
10 15 20
ACT TCA GTG TTA TAT TTA AAA TAGATAAATT TGTGAAATAA CAATTTCTTG 2492
Thr Ser Val Leu Tyr Leu Lys
25 30
GAGACAGTCA ACTTTTTATG ATTTAATCTA AAGATTGTCA TGTACATGTC AACGGATATC 2552
TGAATCCTCA GTGAAACTGT TAAGTTTTCA TTAAATGACT CTGCTGCAAT ACTAGTTTTC 2612
TTCTCAGAAA ATGGAATTCA AATAAAATAA GTTTTTTGGT CTTGGAAAAA AAAAAAAAAA 2672
AAAAAAAAAA AAAAAA 2688

SEQ ID NO:2
LENGTH:1016
TYPE: nucleic acid
STRANDEDNESS: double
TOPOLOGY: linear
MOLECULE TYPE:cDNA to mRNA
ORIGINAL SOURCE:
  ORGANISM:human
  CLONE: 99
FEATURE:
  KEY: CDS
  LOCATION: 231.. 320
  LOCATION: 382.. 483
  LOCATION: 641.. 832
  IDENTIFICATION METHOD: E
SEQUENCE
GAATTCGGCA CGAGAAGAAA CTTAGCCAGG CACTGAGTGC GTGGGAAGGC AAAACACTCT 60
TTTCTCCTCC CACGGCGGGG GCGCTCCCTG GGCGATGCTC ACGGAGAGTG GGAGGGCGTC 120
CCGCACCGGG CAGAGCTGAA GGGTGGCGGG GGTCGGCTTC CTCCTCTCTG CCTCTTCCCT 180
TCCGCCACGC TTCCTACCCA CACCAGCCAG TCTCCAGTAA CAGATATAGA ATG CCT 236
Met Pro
1
TCA TTT GGA ACA CGG AGA GAC AAG CGT GCC GTG TGG GAA GGA GAG AAG 284
Ser Phe Gly Thr Arg Arg Asp Lys Arg Ala Val Trp Glu Gly Glu Lys
        5                   10                  15
CCG CTG CTG GAG CAA GCG TGG TCC CAG AGG AGA GCC TGATTTGAGT 330
Pro Leu Leu Glu Gln Ala Trp Ser Gln Arg Arg Ala
    20                  25                  30
TAAAGCGAGA GGGCAGGAGA AGCAGTTGGG AATCGAGGCA GTGGGCCGAG G ATG GCC 387
Met Ala
1
CAG GTT CAA GAG GCA CAG TGG GAG GCT GCA GGT GTA AGG AGA AGG ACG 435
Gln Val Gln Glu Ala Gln Trp Glu Ala Ala Gly Val Arg Arg Arg Thr
5 10 15
GCT CAG GTG AGG GCT GCA CAG GCG CTG AGG GGA TGG GGC TCT GGG TGG 483
Ala Gln Val Arg Ala Ala Gln Ala Leu Arg Gly Trp Gly Ser Gly Trp
20 25 30
TGACATCTCC AAGGCAAGAG AGCACGTAGA TTTCAGAGCA AAACTATCTG GGTCAGAATC 543
CCGGATCCAC CCTTTATCAC TATAAAATGT TGGGCAAGTA CTCACAGTGC CTCTGCTCTC 603
TCATGTATAA AATGTGAGGA ACCATAGCAC CAAAATC ATG GAG GTG TTA GGC AGA 658


1               5
TGG ATG TGC TGG GAG GAA ACC TGT TTA CTA GGG GGC TGG GTT GGA GAT     706

            10                  15                  20
GGG GAG CTT CTT ATG ATG TCT GCT GTC AGC AGC TTT AAA GGA GAT GTG     754

        25                  30                  35
GGT GTC TAT CCT GGT GGT CTC CCT ATC AGC CTG GGA TCA GGT GGT GAG     802

    40                  45                  50
TGG CTT CAC CTA CTA AGC CCA AAG ATG TGT TAGGAAAACA GGCCGATATC       852

55                  60
TATCGATAAG GAAAACTGGC CGGCACGATG GCTCATGCCT GTAATCCCAG CACTTTGGGA   912
GACCAAGGCG GGCCGATCAC CTGAGGTCAG GAGTTCGAGA CCAGCCTGGC CAACATGGCA   972
AAACCCTGTC TCTCCTAAAA ATACAAAAAA AAAAAAAAAA AAAA                   1016

SEQ ID NO:3
LENGTH:1420
TYPE: nucleic acid
STRANDEDNESS: double
TOPOLOGY: linear
MOLECULE TYPE:cDNA to mRNA
ORIGINAL SOURCE:
  ORGANISM:human
  CLONE:198
FEATURE:
  KEY: CDS
  LOCATION: 378.. 575
  LOCATION: 590.. 709
  LOCATION: 1105.. 1206
  IDENTIFICATION METHOD: E
SEQUENCE
GAATTCGGCA CGAGCTGGAG AGAAAGCTTA TAAATGTGAA GAATGTCACA AAGTTTACAG     60
TCGCACATCA AACCGTGAAA GACAGGAGAA TTCACACTGG AGAGAAACCA TAAAAATGTA    120
AGAGTTTGTG ACAAGGCTTT TGGGCATGAT TCGCACCTGG CACAACATGC TAGAATTCAC    180
ACTGGAGAGA AACCTTACCA GTGTAATGGG TGTGGCAAAG CCTTTAGTAG GCAGTCAACA    240
CTTGTTTACC GTCAGGCAAT CCATGGTGTA GGGAAACTTT ACTAAGGTAA TGATTGTCAC    300
AAAGTCTTCA GTAATGCTAC AACCATTGTG AATCACTGGA GAATCCATAA GGAAGAGAGA    360

TCATACTAGG GTAATAA ATG TGG CAG ATT TTT CAG ACA TTG TTC ATA CCT 410
Met Trp Gln Ile Phe Gln Thr Leu Phe Ile Pro
1               5                   10
TGC AGT TCA TCG GTG AAC TCA ACG CTG GAG AGA AAC CTT ACA AAT GTC 458
Cys Ser Ser Ser Val Asn Ser Thr Leu Glu Arg Asn Leu Thr Asn Val
            15                  20                  25
ATG ACT GTG GCA AGG TCT TCA GTC AAG CTT CAT CCT ATG CAA AAC ATA 506
Met Thr Val Ala Arg Ser Ser Val Lys Leu His Pro Met Gln Asn Ile
        30                  35                  40
GGA GAA TTC ATA CAG GAG AGA AAC CTC ACA TGT GTG ATG ATA GTG GCA 554
Gly Glu Phe Ile Gln Glu Arg Asn Leu Thr Cys Val Met Ile Val Ala
    45                  50                  55
AAG CCT TCA CTT CAC ACC TCA TGAGACATCA GAGA ATG CAT ACT GGA CAG 604
Lys Pro Ser Leu His Thr Ser Met His Thr Gly Gln
60 65 1 5
AAA TCT TAC AAA TGT CAT CAA TGT GCC AAG GTC TTC AGT CTG AGT TCA 652
Lys Ser Tyr Lys Cys His Gln Cys Ala Lys Val Phe Ser Leu Ser Ser
10 15 20
CTC CTT GCA GAA TAT GAG AAA ATT CAT TTT GGA GGT AGT TGG TCC ATA 700
Leu Leu Ala Glu Tyr Glu Lys Ile His Phe Gly Gly Ser Trp Ser Ile
25 30 35
TGC AAT GAG TAGAGCAAAC CATCAAGCAT TAATTGACAT TAGGGTCAAT 749
Cys Asn Glu
40
TCAGCATTGA CTTGAGTTTG TATTGACTTA ACATTGAGTT CAAGCATTAA TTGACATTAG 809
TGTTTATGTT AAGAGGATTG GGCCAGGCAC ATCAGCTTAC ACCTGTAATC TGAGCGCTTT 869
GGGAGGCCAA GGTGGGTAGA TCACTTGAGG TCAGGAGTTT GAGATCAGCC TGGCCAACAG 929
ACGTGAGCCA TTTTCCCAGC CTGTTTTTTG TTTCTTTAAA AAAACTGATA GGGATTTTTA 989
TGGATATCAT GTTGAATCTA AATCACATTG GGTTATTATA TAATCATTTC ACAATATTAA 1049
TTTTTCCAAG CTATCAATAT GGGTTGTAGC TCAATGTTTT TAATCATTTT GATCA ATG 1107
Met
1
TTT GTA GAT TTC AAG GTA CAA ACT TCT GAC CTT TGT ACG TTT ATT TCT 1155
Phe Val Asp Phe Lys Val Gln Thr Ser Asp Leu Cys Thr Phe Ile Ser
5 10 15

AAG TAT TTC TTT AAG TTC TCC AGC AAA TGG AAG TGT TTT AAA ATT TTC 1203
Lys Tyr Phe Phe Lys Phe Ser Ser Lys Trp Lys Cys Phe Lys Ile Phe
20 25 30
TTT TAAAATTGTT TATTGTTAAA GTATGGAAAT TCAACTAATT TTTGGTGCTG 1256
Phe

ATACTGTATT GTGCAAATCC ACTGACTATG TTACTTAGTT CCAGTAGTAT TTTGGTTGGC 1316
TCTTTGTGAT TTTCTACACA GAAGATTATG TCATCTACAA ACAAATATAA TTTTACTTCT 1376
TAAAAAAAAA AAAAAAAAAA AAAAAAAAAA AAAAAAAAAA AAAA 1420

SEQ ID NO:4
LENGTH:1278
TYPE: nucleic acid
STRANDEDNESS: double
TOPOLOGY: linear
MOLECULE TYPE:cDNA to mRNA
ORIGINAL SOURCE:
ORGANISM:human
CLONE:328
FEATURE:
KEY: CDS
LOCATION: 166.. 261
LOCATION: 704.. 829
IDENTIFICATION METHOD: E
SEQUENCE
GAATTCGGCA CGAGGACTGC AATTGGAGTA TTTCATGGTA TGGCTTGATA AAATAAGTTT 60
TAACTACCTT ATCCATAATA CACTCTGATC CTCAAATAAG TAAAATTTTA ATCCATGTGT 120
TTTGAAAACC CATGGATTAA ACACACAGAT TAGGAAGGAG TACCT ATG GAA TGT GAA 177
Met Glu Cys Glu
1
GAC CTG GAA ACG GAT TGC CTA AGA GTT ATC CAA GCA AAA AAA GAA AAA 225
Asp Leu Glu Thr Asp Cys Leu Arg Val Ile Gln Ala Lys Lys Glu Lys
5                   10                  15                  20
AAA AAA AAA AAG AAT TAT CCA AGC ATA TGC ACT CCT TAGAGATTCC 271
Lys Lys Lys Lys Asn Tyr Pro Ser Ile Cys Thr Pro
                25                  30
TCGTGCCCCA AAGTGAAGAC TTCCATACTA TACCATGCCA AAGAGCTCCT GGATAAGTTT 331
TCCTGACTCA TAACACAACT GTTCTTCCTA TTCTTCTGTT TCAAAGAGGA AACAGAGCAT 391
TTTAGTATAT TTGAAAGAAT ACACATCACT AACGCCTGGT GCTTCAGTGT TTCCAAATCA 451

CCATGCTTTC CAACATGGGC TAAAATACAG TAGCCCCCAT TACGTGTGGG GGATATGTTC 511
TAAGACCCCC AGAGGATGCC TGAAACCACA GATAGTATCA AACCCTATAC AGCCCATCTT 571
TTCCTATATG TATGTGCATA GATACCCATG AGGAAGTTTA ATTTATAAGT TGGGCACAGC 631
AAGAGATTAA CATTAATAAC TAATACTAAA ATAGAACAAT TAAAACAATA TATTGTAATA 691
AAAGTTATGT GA ATG TGC TCT CAA AAA TAT CTT TTT TTT GTT TTG AGA CGG 742
Met Cys Ser Gln Lys Tyr Leu Phe Phe Val Leu Arg Arg
1 5 10
AGT CTC ACT CTC ACT CAG GCT GGA GTG CAG TGG CAC AAT CTC GGC TCA 790
Ser Leu Thr Leu Thr Gln Ala Gly Val Gln Trp His Asn Leu Gly Ser
15 20 25
CCA CAA CCT CTG CCT CCT GGG TGT GGT GGT GGG TGC CTA TAATCCCAGC 839
Pro Gln Pro Leu Pro Pro Gly Cys Gly Gly Gly Cys Leu
30 35 40
CACTCAGGAA GCTGAGGCAA GAGAACTGCT TGAACCTGGG AGGTGGAGGT TGCAGTGAGC 899
CAAGATCGTG CCATTGCACT CCAGCCTGGG CAAATAGAGT GAAACTCCGT CTCAAGAAAT 959
AAAAAAGAAA AGCCCAGGTG CAGTGGCTCA TGCCTGTAAT CCCAGAACTT TGGGAGGCCA 1019
AGGCGGGTGT ATCACTTGCA GTCAGGAACT CAAGACCAGC CTGGACAACA TGGTGAAACC 1079
CCATCTCTAC TAAAAATACA AAAAAATATC CAGGCGTGGT GGTGGGCGTC TGTAATCCCA 1139
GCTACTTGGG AGGCTGAGGC AGGAGAATCG CTTGAACCCA GGAGTCAGAG ATTGCGGTGA 1199
GCTAAGGTCG TGCCACTGCA CTCCAGCCTA GGCAACAAAA GCGAACCTCC GTCTCAAAAA 1259
AAAAAAAAAA AAAAAAAAA 1278

SEQ ID NO:5
LENGTH:1242
TYPE: nucleic acid
STRANDEDNESS: double
TOPOLOGY: linear
MOLECULE TYPE:cDNA to mRNA
ORIGINAL SOURCE:
ORGANISM:human
CLONE:335
FEATURE:
KEY: CDS
LOCATION: 593.. 742
IDENTIFICATION METHOD: E
SEQUENCE
GAATTCGGCA CGAGCCTTTT TGTAACCCTG AGCCATAAAT TCCCTTTCTT TCTCATTTAA 60
ACACACTGAA GTACCATCTA AGTATGTGAG AACTGTGAAA GGAGCACCTG TCACAACACC 120
TAAGTCCTTA ATGCTTTGCT CATCCAAGTT GGAGTGTACT AATAATGTGA TTTTTCACAT 180

AATAATGGCA AAAATAAGTA AAAAGTAGCT TAGGAGGTGC AGAGAGACTT ATAAAAAGAA 240
GTATAACCAC CTATGGCACC AAATATCAAA CAGATTACAT AAAAACCATT TTTCTTATAA 300
TCCGTGTCTT TTTTTCTGGG TTTTTCTCTT GCTCTGGTTT GGTGGACTGA CTTTAAGCTG 360
ACCTCCAGTC CTTATGCCCT CACGTAATTC CCTTGTGTTG AGTGTGGGCT GGTCTGGTTC 420
TAACCAATAG AACATACCAA AGGTTATGGA TTGTCATTTT CATGACTAGG TTGCAAGAGA 480
CAGTAACTTC TGGTTAGCTA GTAGACTCCC TTGCTGGCTT TTATGAAGGA TACTGCTATG 540
TTGTGAGTGA TCCTATGGAG AAGTCCATGT GATAAGGGAC TGAGGGTAGC TT ATG GCT 598
Met Ala
1
GGC AGC CAA CAA GGA ACT AAG ATT CTC GGT TCA AAA CAT AGA AAA ACC 646
Gly Ser Gln Gln Gly Thr Lys Ile Leu Gly Ser Lys His Arg Lys Thr
        5                   10                  15
AAA TCC TGC CAA CAC TTG TGT GAC CTC ACT TTC TGG CTG AGC CTT TGG 694
Lys Ser Cys Gln His Leu Cys Asp Leu Thr Phe Trp Leu Ser Leu Trp
    20                  25                  30
ATG ATA TGC ACC TTG CCC ATC GCC TTG ATT ATA GCC TGT GAG AAA CCC 742
Met Ile Cys Thr Leu Pro Ile Ala Leu Ile Ile Ala Cys Glu Lys Pro
35                  40                  45                  50
TGAAGCAGAA ACCCCAGCTA AGATACATCT GAACTCCTGA CCAAAAGAAA CTGTGAGATA 802
ATAAATACGT GTTGTTTTAA GATGTTAAAT TTGTGGTAAT TTGTCCTGCT GTAATAGAAA 862
TAATTTTAAA ATTGTTTTAG GGAAGATCTG TGAGTGGCAC ACTGATCTTT TGTGAGGCTA 922
AAAAGTATTT TTTTGGCCGG ATGCAGTGGC TCGTGCCTGT AATCCCAGCA CTTTGGGAGG 982
CCAAGACGGG CGGATCACTT GAAGTCGGGA GTTCGTGACC AGCCTGGCCA ATATGGTGAA 1042
ACCCCGTCTC TACTAAAAGT ACAAAAATTG GCTGGGCATG GTGGTGCGTG CCTGTGGTCC 1102
CAGCTGCTTG GGAGGCTGAG GCGGGAGAAT CACTTGAAAC TCCGAGGCGG AACTTGCAGT 1162
GAGGCGAGAT CAAGCCACTG CACTCCAGCA TGGGCAATGA GCGAGACTCC GTCTCAAAAA 1222
AAAAAAAAAA AAAAAAAAAA 1242

SEQ ID NO:6
LENGTH:1342
TYPE: nucleic acid
STRANDEDNESS: double
TOPOLOGY: linear
MOLECULE TYPE:cDNA to mRNA
ORIGINAL SOURCE:
ORGANISM:human
CLONE:547
FEATURE:

KEY: CDS
LOCATION: 591.. 719
IDENTIFICATION METHOD: E
SEQUENCE
GAATTCGGCA CGAGAAGATG ATTCAAGATT TGTGCCGGAC AAGGAGAAAA AGAAAATTTG 60
TTGATATCTT TGGGTGAGGA TGGAGAGGTC CAAGAAACCA TTTTGCAAAT GTTTTATATT 120
ACTAACAAGA CAATCTAAAC CTAAATAAAC ATCTTTTTCA GAGAAAAGAC ACTAGAGAAA 180
GATTTAATTT GTGATATTTC AAGCTAGATT ATAAACTGTG ATCTTTGTGA ATGAATTAAT 240
TCATTCCAGA ATTAAATTTT GGTCTTTGTA TTTTGATTCC TTTTACTTTA GTGAGGATTT 300
TGTAGAGAAG ATGAAATTTC ACCTCTATAA TAATAGAAGT TAGGAGTGAG GTCTCACAAA 360
CATGGGCTGT CTTCTAGATT TGGGATATAC CATCAGAGAC AATAAATAAC TGAAATTTGG 420
AAACTGGATA AAAGAGTCTG CTAGTTAGGA CCACTTGACT AAGAGTGGGT ATAGAAGAAA 480
CCTAAGGAAG AAAAGTTAGA TTTGAGACAA CATATTGGAG TATGTTGAAA TTAAAGGTGA 540
ATAAAGTAGT GGAAATTCGA TAGAATTTAA AGGAGACATT GTAGTGGGAG ATG GTA 596
Met Val
1
GAG GTG ATT AGA TAT AAT ATG TAT ATT TAT TTG AAA AAT AGA TAT ATA 644
Glu Val Ile Arg Tyr Asn Met Tyr Ile Tyr Leu Lys Asn Arg Tyr Ile
        5                   10                  15
AGA ATG AAA GTT GTA TGT AGT ACC AAC AAG TAC CAT TCG TAT TAT GAT 692
Arg Met Lys Val Val Cys Ser Thr Asn Lys Tyr His Ser Tyr Tyr Asp
    20                  25                  30
TCA CAA TTT ACA GAG TAT TTT CAT ATA TAAAAATCTT GTGCAATGGG 739
Ser Gln Phe Thr Glu Tyr Phe His Ile
35                  40
TTGTTTTTTC ATTTTATAGA AGGAGCAGAG GCTAAAGGAA GTTGAGGTTA AGTGATTTGG 799
AAGTTACAAA AACTAGTAAG AGCTCAAACT AGCAATAAAA TTATAGTATG TTTTTCCCTT 859
GCCTTCACTG TAATGCTTAA TGGTTGTGTA GTCTTATACG TGACTCCTGA CTTCAAGGAT 919
CCTGGTCTGT ACCTCTTTAG GTCAACACGT TTTGAGTGAA CTGGTGTTGG TTATTTGGAA 979
TTAGATATAA AGTCATATAT TCTTTGGTGA GGAATGGCTT CATATAGGAG TTCACATTCA 1039
AAACAAGCTT TGACAAAATA ATAGAGTGAA AATTGGTAGA TCAGAGTTGA GCTGATTGGA 1099
GGACCAAATT AAAAGACTGG CTGGGCATGA TGGCTCACAC CTGAAAACCC AGCACTTTGG 1159
GAGGCCAAGG CAGGCAGATT GTTTGAGCCC AGGAATTCAA GACCAGCCTA GATAACCTGG 1219
GTATCCCAGC TACTTGGGAG GCTGAGCTGC CGAGGCTACA GTGAGCCATG ATCGTGCCAT 1279
TGCTTTCTAG CCTGGGTGAC AGATTGAGAC CCTGTCTCAA AAAAAAAAAA AAAAAAAAAA 1339
AAA

1342

SEQ ID NO:7
LENGTH:3527
TYPE: nucleic acid
STRANDEDNESS: double
TOPOLOGY: linear
MOLECULE TYPE:cDNA to mRNA
ORIGINAL SOURCE:
ORGANISM:human
CLONE:998
FEATURE:
KEY: CDS
LOCATION: 140.. 1084
IDENTIFICATION METHOD: E
SEQUENCE
GAATTCGGCA CGAGTAGATA TTCTCTACGT CAAAGACTTG GCCTTTGTGG ACCCCGATGA 60
CTGCACCCCC CTCAAGACTA TCACTCGCTT CTATAACCAC CCGGTGCACT TTGTCTTCCA 120
TGACACCAAG TTGGATGCC ATG CTG GAG GAG TTC AAG AAG GGG AAG TCC CAC 172
Met Leu Glu Glu Phe Lys Lys Gly Lys Ser His
1               5                   10
CTG GCC ATC GTG CAG AAG GTA AAC AAC GAG GGT GAG GGT GAC CCC TTC 220
Leu Ala Ile Val Gln Lys Val Asn Asn Glu Gly Glu Gly Asp Pro Phe
            15                  20                  25
TAC GAG GTC CTG GGC CTG GTC ACC CTG GAG GAC GTG ATC GAG GAG ATC 268
Tyr Glu Val Leu Gly Leû Val Thr Leu Glu Asp Val Ile Glu Glu Ile
30 35 40
ATC AAG TCG GAG ATC CTG GAC GAG TCC GAC ATG TAC ACT GAC AAC CGA 316
Ile Lys Ser Glu Ile Leu Asp Glu Ser Asp Met Tyr Thr Asp Asn Arg
45 50 55
AGC CGG AAG CGG GTG TCT GAG AAG AAC AAG CGT GAC TTC TCT GCC TTC 364
Ser Arg Lys Arg Val Ser Glu Lys Asn Lys Arg Asp Phe Ser Ala Phe
60 65 70 75
AAG GAT GCG GAC AAT GAG CTC AAA GTG AAA ATC TCC CCG CAG CTC CTC 412
Lys Asp Ala Asp Asn Glu Leu Lys Val Lys Ile Ser Pro Gln Leu Leu
80 85 90
CTG GCC GCT CAT CGC TTC CTA GCC ACA GAG GTC TCT CAG TTT AGC CCC 460
Leu Ala Ala His Arg Phe Leu Ala Thr Glu Val Ser Gln Phe Ser Pro
95 100 105

TCC CTG ATA TCA GAG AAG ATC CTG CTG CGG CTA CTC AAG TAC CCA GAT 508
Ser Leu Ile Ser Glu Lys Ile Leu Leu Arg Leu Leu Lys Tyr Pro Asp
110 115 120
GTC ATT CAG GAA CTC AAG TTT GAC GAG CAC AAT AAG TAC TAC GCC CGC 556
Val Ile Gln Glu Leu Lys Phe Asp Glu His Asn Lys Tyr Tyr Ala Arg
125 130 135
CAT TAC CTG TAC ACC CGA AAT AAG CCG GCC GAC TAC TTC ATC CTC ATC 604
His Tyr Leu Tyr Thr Arg Asn Lys Pro Ala Asp Tyr Phe Ile Leu Ile
140 145 150 155
CTG CAG GGG AAG GTG GAG GTG GAG GCA GGG AAG GAG AAC ATG AAG TTT 652
Leu Gln Gly Lys Val Glu Val Glu Ala Gly Lys Glu Asn Met Lys Phe
160 165 170
GAG ACG GGC GCC TTC TCC TAC TAT GGG ACT ATG GCC CTG ACC TCG GTC 700
Glu Thr Gly Ala Phe Ser Tyr Tyr Gly Thr Met Ala Leu Thr Ser Val
175 180 185
CCC TCC GAC CGT TCC CCA GCA CAC CCC ACC CCA CTC AGC CGC TCA GCC 748
Pro Ser Asp Arg Ser Pro Ala His Pro Thr Pro Leu Ser Arg Ser Ala
190 195 200
TCC CTC AGT TAC CCA GAC CGC ACA GAC GTC TCA ACT GCA GCA ACC TTG 796
Ser Leu Ser Tyr Pro Asp Arg Thr Asp Val Ser Thr Ala Ala Thr Leu
205 210 215
GCA GGC AGC AGC AAC CAG TTT GGC AGC TCT GTC CTG GGC CAG TAC ATC 844
Ala Gly Ser Ser Asn Gln Phe Gly Ser Ser Val Leu Gly Gln Tyr Ile
220 225 230 235
TCT GAC TTC AGC GTC CGG GCA CTC GTG GAC TTG CAG TAC ATC AAG ATC 892
Ser Asp Phe Ser Val Arg Ala Leu Val Asp Leu Gln Tyr Ile Lys Ile
240 245 250
ACT CGG CAG CAG TAC CAG AAC GGG CTG CTG GCT TCT CGC ATG GAG AAC 940
Thr Arg Gln Gln Tyr Gln Asn Gly Leu Leu Ala Ser Arg Met Glu Asn
255 260 265
AGC CCT CAG TTT CCC ATA GAC GGG TGC ACC ACC CAC ATG GAG AAC TTG 988
Ser Pro Gln Phe Pro Ile Asp Gly Cys Thr Thr His Met Glu Asn Leu
270 275 280
GCC GAG AAG TCT GAG CTG CCT GTG GTG GAC GAG ACC ACA ACT CTT CTC 1036
Ala Glu Lys Ser Glu Leu Pro Val Val Asp Glu Thr Thr Thr Leu Leu

285                 290                 295
AAC GAG CGT AAC TCC TTG CTG CAC AAA GCC TCC CAC GAG AAT GCC ATC    1084
Asn Glu Arg Asn Ser Leu Leu His Lys Ala Ser His Glu Asn Ala Ile
300                 305                 310                 315
TGACAGGAGG GCCCGGGGCC CCCTGCCCAC CCTGCGGGGG CCTCCCCAGT GGGCCCACAT    1144
GAAGAGAGGG AACCTGTTAG TCCAGAAAGG ATACGGATAG ATAGCCTGTC TGACTGAACA    1204
GCCAGATGGC CCCCAGCCTA TGGGGGATCT GGCCTCTGCC AGGGACCTCT GAGTAGCTCT    1264
GAGGTGGCAC TGTCCAGCCC TGGATAGGGG GGGCAGTGGG CCAGCTACCG TAAGCAAAGG    1324
CTGTTTTTTA CTGAGAGAAT TTCTAAAGTA GGCTCATCAC TTTTTTTTAA ATATCATTTT    1384
GGGAAGGGAA GACAGGGTTA AGGAACTTTA TTTAAAAAAA AAATATTTTT TTCCTAAAAA    1444
CTATAAAAGA GGAAGGGTTT CTTGTCCCGG GAAGCAACGG ACATAATCTG TTCCCAGCCA    1504
TGGCCTTCCA GCTTGTGTCC CTGATTCAGG GAGCTCTCCC TTCCTCCTCC TCCTCCTCCT    1564
CCGGAGGTGG GATCCCAGAG CCTGCCAGTG GAGGCTTATC TGTTGGGAGG AAGACAGCTC    1624
TTCACAGAAG CAAAGAACAA AATGGCATGG AGATCAGCTG CCTGAGCACC TGCGCTGTAG    1684
CTTATCTGAC AACGCTGAGG CCACGAGCTC CTGGGTAGCT GTGATCAGGG ACATGATAAT    1744
CTGAGCTATG CAGAGGAGCA CATCTGTTGT CAACTGCTGT ACCCAGAAAT CTAGAACTCT    1804
GCCGACAGCC TCTCCTGGTG AGTCGGGACT CAGCTGAGGA CACATCCCCA CCCTGCCTCC    1864
CATCTGGCCC TTTGGACAAC TGGCCCTTTG TGACAGGGCT GACTCAAGTG TTAGGCAGGG    1924
TCTCAGGCCT TTGATTGCTC ACCCCTGCTC CCCAGGCCCT GCCCTCACTT TTACCAAAGG    1984
TTCTCCCTCG GCGGGAGGGC ATCTGTGTTG GAGGTGATTT GTCTGGGTTC TTCCTTTTGG    2044
TTCCAGAAGG AACTGTCAGT CATCAGCATC TGCGTTGTTA GCAGTCAGTA CCACCCCCGC    2104
CCCACAATGA CAGTCAAGGC TGACTTGTTG ACTGAAGCCT TTTTCCCAGA CCCCTTATTT    2164
CGAATCCCCA AGCTTCAGTC CCTCTTGGGG GTGGAGACAA GAGGACATGT GGGAAGCCAC    2224
GGAAGCAGGT TCTTTATGTC CTCTCCTCTG TGGCTGGCAA GGCTCACCTG GCCTTATCCA    2284
CCCACTTATG GAACCTCAGG AGAGGAGGGC TCCTCCTAAA GGCATGCAGC TTGCAGCCCC    2344
TCTTTCTCAC ACGTGTGATC CTAGCGTGAG AGGTCATCCT GCCCTTGCTG AAGTTAGTAG    2404
TACTGTACTA AGAGCTCTGC CCTCATGTGA ATTCCTGCCC TGGCGCCTCT TCCCTGGGGC    2464
TGAATCAGGC CCTGCTGCAA AACTCCAGGC TTCCCAGGGT TGGGGAGGCT GTGGGACCAA    2524
GGTCCATGTT GGTCCTTCCA CTGGGTGCAG CAGGAGCTGG GTCCCGAGAG CCTGGCAGGT    2584
GAAACTCTGC AGGCCTTCCG CCTGATTATT ATTTATTCAC TCCTTTCCTC ACCCCAAGTG    2644
CCCTGCTCTC CAGGTGCCTA GAGTATCCTA ACTCTTAGGA CCAGGGATTG TCTTGCACCA    2704
AGTATGCCTA CCCCTGGCCA GTCTGAGGTC TCCTAGCCAT AGAACTGACT CCTGGAAGCC    2764
TGGAGAGAAG GTGGTGACAC CCATGGGTTC TCAACTGTAA GGAAAAAAGA CACCAGACTT    2824
TTGTTCCCTA GTGGGGGAAA GCCCTTAGTC TTGTACAGGA GCAGCTTGCT CCCAAGTCCT    2884
TTTGGAAGCT GGCAGAGCTA TATTCCTGAC AGCCCTGACT GCCAGGTAGA GCAAAAGACA    2944

TTGGTGGGGG TATGTGAAGC AAAAGGGGCA GGTGCACACA CCTCCACAGT GACCTCTGTG 3004
CACACGGTTA CCACCAACTG GCTGGCCCTC CTCCTCTTCC CTGGCCCATT GATCATCCCT 3064
TCTCACAGAG GGTCATCATT ATTTCCAAAT ATTGTTTGTC TGATGACTTC CTCTTCCCAG 3124
TGCAATTTTT CCCTTCCTAT TTCAACCTCT GGTTCCTGGG ATGAGCCATA CCCTGGAACT 3184
GGCCCACCCA CTGTGTCTTC CATGTAAGGG AGACCTTTGC AAAGGGCATC CAAATGGGTA 3244
GGCAGGTGAC AGCCGCCGTA TTTATTTTGC ATAATATTTT AATTTGTATA TTTTTGTGAT 3304
TTATTTTGGC GTTATGAGTT TGACTCTCGG GGAGTTTTGT TGTTATGACT CTTGTGTCTT 3364
TTGTCACAAA ACAATGATAT TTGCTAAACG ATATATGGAA TTTATTTTTG ATTGGTAATA 3424
AAAAATCAAA TATGTATAAA TCCTGGTGAA TCTAAAAAAA AAAAAAAAAA AAAAAAAAAA 3484
AAAAAAAAAA AAAAAAAAAA AAAAAAAAAA AAAAAAAAAA AAA 3527

SEQ ID NO:8
LENGTH:1512
TYPE: nucleic acid
STRANDEDNESS: double
TOPOLOGY: linear
MOLECULE TYPE:cDNA to mRNA
ORIGINAL SOURCE:
ORGANISM:human
CLONE:1148
FEATURE:
KEY: CDS
LOCATION: 78.. 155
LOCATION: 618.. 725
LOCATION: 884.. 1018
IDENTIFICATION METHOD: E
SEQUENCE
GAATTCGGCA CGAGGAGAGA AAGAAAAAGA GAAAGAAAAA AAGAAAGAGG AAGGAAAGAA 60
GGAAGGAAGG AAGGAAA ATG GAG GCA TTA CAG CTG ATA CCA CAG GAA TAC 110
Met Glu Ala Leu Gln Leu Ile Pro Gln Glu Tyr
1               5                   10
AAA AGA TTA TTT AAG ACT ACT ATG AAC ACC TCT GTG CAC ATA AAC 155
Lys Arg Leu Phe Lys Thr Thr Met Asn Thr Ser Val His Ile Asn
            15                  20                  25
TAGAAAATCT ATAGGGAATT AATAAATTCC TGGAAACATA CCACCTCCCC AGCTTGAATC 215
AGAATGAAAC AGAAATTCTG AACAGACCAA CAACAAGCAG TGAGATTGAA TCAGTAATTT 275
AACAAAGTTG TCCTCCTAAA AAAAGCCAAG GGCCATACAA ATTCACAGCC AAATTCTACC 335
AGACATTCAA AGAATTGGCG CCAATTTTGC TGAAACTATT CTAAAAGACT GAGAAAGAAG 395
GAATCCTTCC TAACTCATTC TATGAAGCCA GTATCACCTT GATACCAAAG CCAGGAAAGG 455
GCATACAAAA CAAAACAAAC AAAAAACAAA AAAACACAAA ACGACAGACC AATATTCCTG 515

ATTAACATAG ATGCAGAAAT CTTCAACAAA ATACTAGCAA ACTAAACCCA ACAGCACATC 575
AAAAAGGTAA TTAACTATGA TTAAGTGGGT ATTATCCCAG GG ATG CAG GGA TGG 629
Met Gln Gly Trp
1
TTC AAC ATA TGC AAG TCA ATA AAT GTG ATT CAC CAT GTA AAC AGA ATT 677
Phe Asn Ile Cys Lys Ser Ile Asn Val Ile His His Val Asn Arg Ile
5 10 15 20
AAA AAC AAA AAC CAT ATG ATT ATC TCA ATA GAT GCA AAA AGC ATT GGA 725
Lys Asn Lys Asn His Met Ile Ile Ser Ile Asp Ala Lys Ser Ile Gly
25 30 35
TAAAATACAG CATCCTTTTA TAAAACCCTC AACAAATTAG ATATAGAAGG AACATATCTC 785
AAAATAATAA AAGCTACATA TGGAAAACCA CAGCCAACAT CATACAGAAT AGGAAGAAGT 845
CAAAAGCATT CCCCCCTAAG AACTGGAATA AGACAAGG ATG CCC ACT TTT ACC ACT 901
Met Pro Thr Phe Thr Thr
1 5
TCT ATT CAA TAT AGT ACT GGA AGT CAT AGC CAG GCC AAT CAG TCA AGA 949
Ser Ile Gln Tyr Ser Thr Gly Ser His Ser Gln Ala Asn Gln Ser Arg
10 15 20
GAA AGA AAG GAA TTC CAA ATC AGA AAA GAG GAA GTC AAA CTA TTT ATA 997
Glu Arg Lys Glu Phe Gln Ile Arg Lys Glu Glu Val Lys Leu Phe Ile
25 30 35
TTT GCC GAC GAT ATG ATT GTG TAACTAGAAA AACCTAAAGA TCCCTCCAAA 1048
Phe Ala Asp Asp Met Ile Val
40 45
AGACTCCTAG ATTTGAGAAA TGAATTCAGT AAAGTCTCAG GTTACAAAAT CCATGTACAC 1108
AAATCAGTAG CTCTGCTGCA TGCCAACAAC GACCAAGCTG AGAATCAAAT AAAAAACTCA 1168
GTCCCTTATA TAATAGCTGA AAAAAAAAAA ACCTAGGAAT ACATTTAACT GGGGAGGTGA 1228
AAGAGCTCTA CAAGGAGGAC TACAAAACAC CACTGAAAGG AATCACAGAT GATACAAACA 1288
AATGGAAATA CGTGTCATGC TCATGGATTG GAAGAATCAG TATCACACAA ATGATCATAT 1348
TGCTCAAAGG AAGCTTCATA TTCACTGAAA TTCCTATCAA AATACCAAAT TATTTTTCAC 1408
AAAATTAGAA AAAAAATCCA AAAATTCATA TGGAACCAAA AAAAAAAAAA AAAAAAAAAA 1468
AAAAAAAAAA AAAAAAAAAA AAAAAAAAAA AAAAAAAAAA AAAA 1512

SEQ ID NO:9
LENGTH:882
TYPE: nucleic acid

STRANDEDNESS: double
TOPOLOGY: linear
MOLECULE TYPE:cDNA to mRNA
ORIGINAL SOURCE:
ORGANISM:human
CLONE:1180
FEATURE:
KEY: CDS
LOCATION: 97.. 195
IDENTIFICATION METHOD: E
SEQUENCE
GAATTCGGCA CGAGCTTAAT CTATGCAGTA CACTACTTCT TTTCAAAACT GCAGATCACG 60
GGAACAGCAA GCACAATTCT GTACTTTGGT TATACC ATG ATA ATG GTT TTG ATC 114
Met Ile Met Val Leu Ile
1               5
TTC TTT CTT TTT ACA GGA ACA ATT GGC TTC TTT GCA TGC TTT TGG TTT 162
Phe Phe Leu Phe Thr Gly Thr Ile Gly Phe Phe Ala Cys Phe Trp Phe
            10                  15                  20
GTT ACC AAA ATA TAC AGT GTG GTG AAG GTT GAC TGAAGAAGTC CAGTGTGTCC 215
Val Thr Lys Ile Tyr Ser Val Val Lys Val Asp
        25                  30
AGTTAAAACA GAAATAAATT AAACTCTTCA TCAACAAAGA CCTGTTTTTG TGACTGCCTT 275
GAGTTTTATC AGAATTATTG GCCTAGTAAT CCTTCAGAAA CACCGTAATT CTAAATAAAC 335
CTCTTCCCAT ACACCTTTCC CCCATAAGAT GTGTCTTCAA CACTATAAAG CATTTGTATT 395
GTGATTTGAT TAAGTATATA TTTGGTTGTT CTCAATGAAG AGCAAATTTA AATATTATGT 455
GCATTTGTAA ATACAGTAGC TATAAAATTT TCCATACTTC TAATGGCAGA ATAGAGGAGG 515
CCACATTAAA TAATACTGAT GAAAGGCAGG ACACTGCATT GTAAATAGGA TTTTCTAGGC 575
TCGGTAGGCA GAAAGAATTA TTTTTCTTTG AAGGAAATAA CTTTTTATCA TGGTAATTTT 635
GAAGGATGAT TCCTATGATG TGTTCACCAG GGGAATGTGG CTTTTAAAGA AAATCTTCTA 695
TTGGTTGTAA CTGTTCATAT CTTCTTACTT TTCTGTGTTG ACTTCATTAT TCCCATGGTA 755
TTGGCCTTTT AAACTATGTG CCTCTGAGTC TTTCAATTTA TAAATTTGTT ATCTTAATAA 815
ATATTATAAA AATGAAAAAA AAAAAAAAAA AAAAAAAAAA AAAAAAAAAA AAAAAAAAAA 875
AAAAAAA
882

SEQ ID NO:10
LENGTH:1837
TYPE: nucleic acid
STRANDEDNESS: double
TOPOLOGY: linear

MOLECULE TYPE:cDNA to mRNA
ORIGINAL SOURCE:
  ORGANISM:human
  CLONE:1243
FEATURE:
  KEY: CDS
  LOCATION: 225.. 389
  LOCATION: 977..1111
  LOCATION: 1588.. 1698
  IDENTIFICATION METHOD: E
SEQUENCE
GAATTCGGCA CGAGCAGAGA TTCTATCACC ACTGTCCGGT CTTTCTTGGA TGATTTTGAG    60
TCAAAGTATT CCTTCCATCC AGTAGAAGAC TTTCCTGCTC CAGAAGAATA TAAACACTTT   120
CAGAGGATAT ATCCCAGCAA AACAAACCGA GCTGCCCGTG GAGCCCCACC TCTGCCACCC   180
ATTCTCAGGT GAAGCCTGGC TTGGTCCCGT TCCTCAGGAA AAGG ATG GAC CTT CTC   236
                                                 Met Asp Leu Leu
                                                  1
TTC TTC TCA GAT GGT CCC TTC CAT TCC CCT GAA ACC TGC ATG AGA GCT   284
Phe Phe Ser Asp Gly Pro Phe His Ser Pro Glu Thr Cys Met Arg Ala
  5                  10                  15                  20
CCT AAC ATG TTT CTC CAA TGC AAT CAA GCC CTA GAC TCC AAA TGT CCT   332
Pro Asn Met Phe Leu Gln Cys Asn Gln Ala Leu Asp Ser Lys Cys Pro
                 25                  30                  35
CCC AGC TCA CCT CCA TCT ATG CAT CTC ATC TCT GGA TTT GGT GAT CAG   380
Pro Ser Ser Pro Pro Ser Met His Leu Ile Ser Gly Phe Gly Asp Gln
             40                  45                  50
ACT CTA TAT TGACAGTAGG ATCTCAAACC CTGCATCCAT CCTTCCTCCA           429
Thr Leu Tyr
         55
GCAAGCCCTG CTAGCCACAT GAGGAACAAG TTTCCGTGTC TTCTGCCTTC CTCTTGGGGA   489
AAGGTGCCTT GTTGTGATGA ATTAACTCAC TGTTAGGGCA GGGTGGAGAA TGGTACTCCT   549
TCCTTCTCCT GTCCACTGTG GGGGAAGCTT GGCAGGTATA TTATATTTCA TCATTTAGGA   609
GGCTGGCATG ACCAGGACTT ATGGGTGGGA GGGGAGCATT TTTAGTGAAG CAAGAAAGGA   669
GTTTGCCAAG AAGTGATCTG TTTTAAAGGT CATATTTGGA GAAAGGGCAA GGAATTGGGT   729
CTGCTTTATT TTTGGGGGTA TTTTGTTTTT GTTCTCACCT GCTGCCCCCC CACCCCACCA   789
CCCCAGGGAT AAATTGGATA TAAACACTAA ATACTAATCA GTTGAACTTA ACATTTAATA   849
AAAAGAAAGG GTGAAATAAA CTGAAGACCA TTTTAGAACT AGTCAGTTCT CTGCAGCAAA   909
GGGAACAGGA GCCATTTGAA CCCTCTGGGA CCCCTCACCC CACTGCTTCA GGGTGCTAGG   969

CTGAGGG ATG TTT TTC CTC CCC CTT ACC GCC CAT GCC CTT AAA AGA AAA 1018
Met Phe Phe Leu Pro Leu Thr Ala His Ala Leu Lys Arg Lys
1 5 10
GTC ACT TTT TGT GGA GGG CAT CAT TCA TTC CTG ATT CAC AAA CCC CAA 1066
Val Thr Phe Cys Gly Gly His His Ser Phe Leu Ile His Lys Pro Gln
15 20 25 30
AAA CCT CTG GTG GGA GAT AGG AAG ATA GGG CGT GGG CCT GGG CCT 1111
Lys Pro Leu Val Gly Asp Arg Lys Ile Gly Arg Gly Pro Gly Pro
35 40 45
TAACCTCAAT CTTGTGTCTG CCTCAGTCTT TTCTGACTGG CCCTGAAGTT GTCAGTGGCT 1171
CTTTCTGTCC TTCAGCCCCT GGAAGGTGCT CCAGGATAAC AAAGAAGGGC AGGTTGAAGC 1231
CCCTCATGGA AGGAGCTGGC TTTGTGGGGC TGCAAAGGAC TTTTAAGTCC TGCCTGTACT 1291
GAAGTTCACA GCCCACCTGA CTGAGCAGAC TCTTCCTGTT CCTTTCTCTA CCACCCTTGC 1351
CTTCCCAGGA CTGCACGGTT TAACACAGCA GAGTACAGAA GGGTGAAGAA GTGAGCAGAG 1411
GCTTATGAAG ATATTCAGAT ACTCTTCTAT GCCAGGAAGC ACAAAGACTT TGTTGAGATT 1471
TGCCTCAGTT CAGTAGATCT TCCTTGGCAG CCAGCCATAG GTTGTTTCTT TGTCTTCCGG 1531
GTCCTAAAGA GCACAGAGAA AATGGAGGTC CCCAGTCTAG GTAGGAAGCT GATTGG ATG 1590
Met
1
AGG ACT TCT TTT TTT CCG ACA GCA GGA TGG GGC TCT TGG GCT CCA CAC 1638
Arg Thr Ser Phe Phe Pro Thr Ala Gly Trp Gly Ser Trp Ala Pro His
5 10 15
ACC AGA TGC TTT GGT TTT CTA CAA CTG TTG CTA TGT GTA GAG GGT GCT 1686
Thr Arg Cys Phe Gly Phe Leu Gln Leu Leu Leu Cys Val Glu Gly Ala
20 25 30
CAG AGC GTG GCA TGAGAGCAAG GAGACCATGG CTACTCTTTG AAATGGATGG 1738
Gln Ser Val Ala
35
GGAAAATTAG CTTAAAAATT TAATCACGAG ATTGCGCCAC TGCACTCCAG CCTGGGCGAC 1798
AGAGCCAGAC TCCGTCTCAA AAAAAAAAAA AAAAAAAAA 1837

SEQ ID NO:11
LENGTH:1858
TYPE: nucleic acid
STRANDEDNESS: double
TOPOLOGY: linear
MOLECULE TYPE:cDNA to mRNA

ORIGINAL SOURCE:
ORGANISM:human
CLONE:2232
FEATURE:
KEY: CDS
LOCATION: 26.. 1255
IDENTIFICATION METHOD: E
SEQUENCE
GAATTCGGCA CGAGGCCGCG CGAAC ATG GCG GCC GAA ATC CAC TCC AGG CCG 52
Met Ala Ala Glu Ile His Ser Arg Pro
1               5
CAG AGC AGC CGC CCG GTG CTG CTG AGC AAG ATC GAG GGG CAC CAG GAC 100
Gln Ser Ser Arg Pro Val Leu Leu Ser Lys Ile Glu Gly His Gln Asp
10                  15                  20                  25
GCC GTC ACG GCC GCG CTG CTC ATC CCC AAG GAG GAC GGC GTG ATC ACG 148
Ala Val Thr Ala Ala Leu Leu Ile Pro Lys Glu Asp Gly Val Ile Thr
                30                  35                  40
GCC AGC GAG GAC AGA ACC ATC CGG GTA TGG CTG AAA AGA GAC AGT GGT 196
Ala Ser Glu Asp Arg Thr Ile Arg Val Trp Leu Lys Arg Asp Ser Gly
            45                  50                  55
CAA TAC TGG CCC AGC ATT TAC CAC ACA ATG GCC TCT CCT TGC TCT GCT 244
Gln Tyr Trp Pro Ser Ile Tyr His Thr Met Ala Ser Pro Cys Ser Ala
        60                  65                  70
ATG GCT TAC CAT CAT GAC AGC AGA CGG ATA TTT GTG GGC CAG GAT AAT 292
Met Ala Tyr His His Asp Ser Arg Arg Ile Phe Val Gly Gln Asp Asn
    75                  80                  85
GGA GCT GTA ATG GAA TTT CAC GTT TCT GAA GAT TTT AAT AAA ATG AAC 340
Gly Ala Val Met Glu Phe His Val Ser Glu Asp Phe Asn Lys Met Asn
90                  95                  100                 105
TTT ATC AAG ACC TAC CCA GCT CAT CAG AAC CGG GTG TCT GCG ATT ATC 388
Phe Ile Lys Thr Tyr Pro Ala His Gln Asn Arg Val Ser Ala Ile Ile
                110                 115                 120
TTC AGC TTG GCC ACA GAG TGG GTG ATC AGT ACC GGC CAC GAC AAG TGT 436
Phe Ser Leu Ala Thr Glu Trp Val Ile Ser Thr Gly His Asp Lys Cys
            125                 130                 135
GTG AGC TGG ATG TGC ACG CGG AGC GGG AAC ATG CTC GGG AGG CAC TTC 484
Val Ser Trp Met Cys Thr Arg Ser Gly Asn Met Leu Gly Arg His Phe

        140                 145                 150
TTC ACG TCC TGG GCT TCG TGT CTG CAA TAT GAC TTT GAC ACT CAG TAT 532
Phe Thr Ser Trp Ala Ser Cys Leu Gln Tyr Asp Phe Asp Thr Gln Tyr
    155                 160                 165
GCT TTC GTT GGT GAT TAT TCT GGG CAG ATC ACC CTG CTG AAG CTT GAA 580
Ala Phe Val Gly Asp Tyr Ser Gly Gln Ile Thr Leu Leu Lys Leu Glu
170                 175                 180                 185
CAG AAC ACG TGT TCA GTC ATC ACA ACC CTC AAA GGA CAT GAA GGT AGT 628
Gln Asn Thr Cys Ser Val Ile Thr Thr Leu Lys Gly His Glu Gly Ser
                190                 195                 200
GTC GCC TGC CTC TGG TGG GAC CCT ATT CAG CGG TTA CTC TTC TCA GGA 676
Val Ala Cys Leu Trp Trp Asp Pro Ile Gln Arg Leu Leu Phe Ser Gly
            205                 210                 215
GCA TCT GAC AAC AGC ATC ATC ATG TGG GAC ATC GGA GGA AGG AAA GGC 724
Ala Ser Asp Asn Ser Ile Ile Met Trp Asp Ile Gly Gly Arg Lys Gly
        220                 225                 230
CGG ACG CTG TTA CTT CAG GGC CAT CAT GAC AAG GTG CAG TCG CTG TGC 772
Arg Thr Leu Leu Leu Gln Gly His His Asp Lys Val Gln Ser Leu Cys
    235                 240                 245
TAC CTT CAG CTC ACC AGG CAG CTC GTC TCC TGT TCC TCG GAC GGC GGA 820
Tyr Leu Gln Leu Thr Arg Gln Leu Val Ser Cys Ser Ser Asp Gly Gly
250                 255                 260                 265
ATT GCA GTG TGG AAC ATG GAT GTT AGC AGA GAA GAG GCT CCT CAG TGG 868
Ile Ala Val Trp Asn Met Asp Val Ser Arg Glu Glu Ala Pro Gln Trp
                270                 275                 280
TTG GAA AGT GAT TCT TGT CAG AAA TGT GAG CAG CCA TTT TTC TGG AAC 916
Leu Glu Ser Asp Ser Cys Gln Lys Cys Glu Gln Pro Phe Phe Trp Asn
            285                 290                 295
ATA AAG CAG ATG TGG GAC ACC AAG ACG CTG GGG CTA AGA CAA CAT CAC 964
Ile Lys Gln Met Trp Asp Thr Lys Thr Leu Gly Leu Arg Gln His His
        300                 305                 310
TGC AGG AAA TGC GGG CAG GCT GTC TGC GGG AAG TGC AGC AGC AAG CGC 1012
Cys Arg Lys Cys Gly Gln Ala Val Cys Gly Lys Cys Ser Ser Lys Arg
    315                 320                 325
TCA AGT TAC CCA GTC ATG GGC TTC GAG TTC CAA GTC CGG GTT TGT GAT 1060

Ser Ser Tyr Pro Val Met Gly Phe Glu Phe Gln Val Arg Val Cys Asp
330                 335                 340                 345
TCT TGT TAC GAC TCC ATC AAA GAT GAA GAT CGG ACT TCT CTA GCG ACC    1108
Ser Cys Tyr Asp Ser Ile Lys Asp Glu Asp Arg Thr Ser Leu Ala Thr
                350                 355                 360
TTT CAT GAA GGA AAA CAT AAC ATT TCC CAC ATG TCC ATG GAC ATT GCC    1156
Phe His Glu Gly Lys His Asn Ile Ser His Met Ser Met Asp Ile Ala
            365                 370                 375
AGG GGA CTG ATG GTG ACC TGT GGG ACC GAC CGC ATT GTA AAG ATC TGG    1204
Arg Gly Leu Met Val Thr Cys Gly Thr Asp Arg Ile Val Lys Ile Trp
        380                 385                 390
GAC ATG ACA CCT GTG GTG GGC TGC AGT CTG GCG ACT GGG TTT TCT CCG    1252
Asp Met Thr Pro Val Val Gly Cys Ser Leu Ala Thr Gly Phe Ser Pro
    395                 400                 405
CAC TGATCTGAGA GCTGGGCGGC GTCCACACCT AAGAACAGCA GCTCCACCAA    1305
His
410
ATGAAGTCCC TCTCACGCAG CTCCACAGCG CTGTCTCGTG AATGGACAGT AGCCACTTAC  1365
AAACAAATCA ACATTTTTAA AAAGAAAATG TAAAGAGATG GGGTCTTGCC AGGTCTTGGC  1425
CAGGCTGGTC TTAACTCCCG GACTCAAGCA ACTCTCCTGA CTTGGCCTCC CAAAGTGTTG  1485
GAATTACAGG CATGAGCCAC TGCAGTAGCC CTGATTGTTT TAAATAGAAG AAGGTCTTTA  1545
GACAAACTCA ACCAATTGTC AACCAGAAAA TGTTGGAAGT TCCCTCCTAC CCCGCCCGCC  1605
TTTGAGTTGT CCGGCCTTTC TGGACCTAAC CAATGTATTT CTTAAATATA TTTGATTGAT  1665
GTCTCATGGC TCTTTAAATG TATAAAAGCA AGTTGTGCCC CAACCCCGGC CACCTTGGGC  1725
ACATGTTCTC AGGACCTCCT GAGGACTGTG TCCACAGGCC ATGGTCACTT ATATTTGGCT  1785
CAGAATAAAT CTTTTAAAAT AAAAAAAAAA AAAAAAAAAA AAAAAAAAAA AAAAAAAAAA  1845
AAAAAAAAAA AAA                                                     1858

SEQ ID NO:12
LENGTH:1082
TYPE: nucleic acid
STRANDEDNESS: double
TOPOLOGY: linear
MOLECULE TYPE:cDNA to mRNA
ORIGINAL SOURCE:
  ORGANISM:human
  CLONE:3282
FEATURE:

KEY: CDS
LOCATION: 262.. 399
LOCATION: 621.. 713
IDENTIFICATION METHOD: E
SEQUENCE
GAATTCGGCA CGAGCGAACT CCTGAACTCA GGTAATCCTC TCACCTCAGC CTCCCGAAGT 60
GCTGGGATTA CGGGCGTGAG CCACTGTGCC TGGCCTGAGA GGACAAATAT TTTTAAAAAG 120
AAATGATAGT TCTTTATGTA TAGAATGTGG GATGATTTTT TTTTAATTGC CCTCGTTTTC 180
AGAACATGTA TTCAGTTTGA TCAGTGATTT TCAGTCTTGG CACTATTGAC ATTTAGGGTC 240
GGATAATCTC CAGGCTATCC T ATG CAT TGT AGG ATG TTC TGT AGC ACT CCT 291
Met His Cys Arg Met Phe Cys Ser Thr Pro
1               5                   10
GGC CAC TAC CCA CTA GAT AGG TCA GTA ACA CCT CTC CTC ACC CCC ACC 339
Gly His Tyr Pro Leu Asp Arg Ser Val Thr Pro Leu Leu Thr Pro Thr
                15                  20                  25
CTG ATT GTG ACT ACC AAA AAT GTC TCT AGA CAT TGC CAA ATG CCC TCT 387
Leu Ile Val Thr Thr Lys Asn Val Ser Arg His Cys Gln Met Pro Ser
            30                  35                  40
GGG GGG CAA ACG TGACCTCTGA TAAAATTCAC TGTCACTTAT GAAGATTCCT 439
Gly Gly Gln Thr
        45
TAATTATTTA TTTATGCCTT TTATGAACTA GGGTTCCCTT GATGTAAGCT AGGTACAGGT 499
GTTGAATGAA TCCCTTCTGC AGATGTAGTG TATAGCTTGG GCGGTGCCTG ATATATGTTG 559
GAGAACACTG GAATATGATG AAATATTTTA GCACTATTGG GAATTTAATT GAAGGTTCGA 619
A ATG AGC TCT TGT TTT GAA AGT AAA GGT GTA GGC TTT CTG CTG TAT CTA 668
Met Ser Ser Cys Phe Glu Ser Lys Gly Val Gly Phe Leu Leu Tyr Leu
1 5 10 15
AAT CTC TGT TTT CCA CCT CTG CCA CCA CCC CCT CAT GCA TTT GCT 713
Asn Leu Cys Phe Pro Pro Leu Pro Pro Pro Pro His Ala Phe Ala
20 25 30
TGAAAAAGAT CCTTACTAGC TTAGGAATAG TTGAGAATTT CGTTTGAAAT GTTTGACATG 773
ATCTGTCTAT ATCTTGTGTT CAAGGAAAGA TAGTTTTGTT TGTTTGCGTG TTTGTTTGTT 833
TTTGAGAACA GGGTCTTGCT CTGTTGCCCA GGTTGGAGTG CAGTGGCTCG ATCACAGCTC 893
ACTTCAGCCT CCACCTCCTG GACTTAAGAG ATTCTCCCAC CTCAGCCTGT TGAGTAGCTG 953
GGACTACAGA TGTGCATGCC ACCAACACTC TGCTAATTTT TCTACTTTTT ATAGAGATGA 1013
GATTTCACCA GCCTGGGAAA CATGGTGAGA CCCCCTCTCT ACCGAAAAAA AAAAAAAAAA 1073

AAAAAAAAA
1082

SEQ ID NO:13
LENGTH:1505
TYPE: nucleic acid
STRANDEDNESS: double
TOPOLOGY: linear
MOLECULE TYPE:cDNA to mRNA
ORIGINAL SOURCE:
  ORGANISM:human
  CLONE:11037
FEATURE:
  KEY: CDS
  LOCATION: 575.. 703
  LOCATION: 863.. 997
  LOCATION: 1121.. 1345
  IDENTIFICATION METHOD: E
SEQUENCE
GAATTCGGCA CGAGGTAGAA TTGACCCAGT GCTGCCCTGG CAACTTTGTA TATTAGGACA 60
AATTTACATT TCTTACCTTT ATGAGAGGCA CCCTGGTAGG CTAGTGGAGT TACACACAAA 120
GTCTGATCTC AGCTGCACTG TCCAGAAATG CAACACGGTC CAATCAAATA ACATTCTCTG 180
AGCCTGTTTA TTTAGCTGTG AAAGAAGAAT AACATACCCA TCTAAAAAGG CAGCTTATTG 240
TATTTGATTG GTCTTTTATT TTCTATGAAA CTGTGTTTAA CACAGTAATT ATTTTCATTT 300
GTGTACTACA TTTGTGTTGT GTTTTTGGTT TTAGTTTTGT TTTTGAAATG GAGTCTTTTT 360
TTTAGTGGTT TTTTGTTTTG TTTTGTTTTG TTTTGTTTTT GAGATGGAGT CTTTCTATTG 420
CACTCCAGCC TGGGCAACGA GCAAAACTCT GTCTCAAAAT AAAAAAAAGA TTTCTTAAAA 480
TGATATTTTC AGTATTTTAT AGATGATGTG TAAGCAGCAA GCTTAATAGG ATGTTACCCG 540
ACACTTTGCG AGACTGGCAG CTGATTTGAT CCAG ATG TCT CTA ATT CTT TTT TCT 595
Met Ser Leu Ile Leu Phe Ser
1               5
TTT TCT TTT TCT GTT TTT TTT TTT GAC AGA GCC GAG ATC ACA CCA CTG 643
Phe Ser Phe Ser Val Phe Phe Phe Asp Arg Ala Glu Ile Thr Pro Leu
        10                  15                  20
CAC TCC AGC CTG GGT GAC AGA GCG AGG CTC CGT CTC AAA AAA AAA AAA 691
His Ser Ser Leu Gly Asp Arg Ala Arg Leu Arg Leu Lys Lys Lys Lys
    25                  30                  35
AAA AAA TTG TTT TAGCGCTGGG TTTCCCAAGG TGGGAGAGAC AGACCCAGCC 743
Lys Lys Leu Phe
40

TGGAGCTGGC CCCTGGCCTG TGTGCTGACT TCTTGGGGTC CTCAAACCAC TGTATTTTTC 803
TGTTGAGCCT GTACTTGGGG AGAGATCAGT AGCATTTGAG GAAGTAAGAG AAAAGAATC 862
ATG GTA CCT CAG GGT TTC TTT CCC TTT ACT CGC TGG CAG CCA TTG TCT 910
Met Val Pro Gln Gly Phe Phe Pro Phe Thr Arg Trp Gln Pro Leu Ser
1 5 10 15
GTG GGC ACC TCA TGT TTT TCC ACA CTC TAC TGG GCC GTG GAG GTA ACG 958
Val Gly Thr Ser Cys Phe Ser Thr Leu Tyr Trp Ala Val Glu Val Thr
20 25 30
ATC ACC CAG GCC AGT CTC CTC TGC CTG GGA TGC GCC CTC TGAGAGGAGG 1007
Ile Thr Gln Ala Ser Leu Leu Cys Leu Gly Cys Ala Leu
35 40 45
CCTAGCAGGG CAGGCTCCCT CTGGGCATCC CTGGATGCAG CCTCTGGACA CATGCCTCCT 1067
TTAAAGTGTC CGGGTGCAGC TCAGGTTGAG TGGAGGTAGA AGGAGAAACA GAC ATG 1123
Met
1
TTT ACC ACG CGT TTT CCA AAG CTC CTG ATC TTT CCC AAG ATT GTA ACT 1171
Phe Thr Thr Arg Phe Pro Lys Leu Leu Ile Phe Pro Lys Ile Val Thr
5 10 15
GAA AAC TGC TGT CTC TTG TTT TGT TCG TTT TGG GGG TGG TGG TGC TGG 1219
Glu Asn Cys Cys Leu Leu Phe Cys Ser Phe Trp Gly Trp Trp Cys Trp
20 25 30
CTG GGC CAT GCT TGT GAA GTG ATG TGT GTC TCT GAT TTA ACG GAT TCA 1267
Leu Gly His Ala Cys Glu Val Met Cys Val Ser Asp Leu Thr Asp Ser
35 40 45
CTG TTT TCT CTG CTA ATT GAG AGA GCG TTA TTT ACA TTA TTT ATT TGT 1315
Leu Phe Ser Leu Leu Ile Glu Arg Ala Leu Phe Thr Leu Phe Ile Cys
50 55 60 65
TTT GAC ACA AGT GCT TTC AGT GTT TTA TCC TAGCTAATGG CTTCTTAAAG 1365
Phe Asp Thr Ser Ala Phe Ser Val Leu Ser
70 75
GTAATAAAAC CCTTCCAACG TAATTGGTCA GATAAAACTT TTTTTCTTGT ATGCTTAAAT 1425
AAAGCAATTA GTGAAGCACT TCTAAAAAAA AAAAAAAAAA AAAAAAAAAA AAAAAAAAAA 1485
AAAAAAAAAA AAAAAAAAAA 1505

SEQ ID NO:14
LENGTH:232
TYPE: nucleic acid
STRANDEDNESS: double
TOPOLOGY: linear
MOLECULE TYPE:cDNA to mRNA
ORIGINAL SOURCE:
ORGANISM:human
CLONE:427
FEATURE:
KEY: CDS
LOCATION: 17.. 178
IDENTIFICATION METHOD: E
SEQUENCE
CGCACAGTAA GGGAGA ATG TGG GGA CTC AGA GCA GGA GCC TTT GGA GTC AGA 52
Met Trp Gly Leu Arg Ala Gly Ala Phe Gly Val Arg
1               5                   10
GAA GTT TTC CAG AAG GAA CAT CTA AGC AGA GCC CGG TTA GGC AAA GTG 100
Glu Val Phe Gln Lys Glu His Leu Ser Arg Ala Arg Leu Gly Lys Val
        15                  20                  25
AAC TGC CAT GGC GAG GGC CTG GAG ACT TCC AGN AGC GTG ATA ACT GCT 148
Asn Cys His Gly Glu Gly Leu Glu Thr Ser Ser Ser Val Ile Thr Ala
    30                  35                  40
CTT AAT TGT TCA ANC CTC ACC AGG AGC TAAGCCCTGT AAGGTGGGTG 195
Leu Asn Cys Ser Ser Leu Thr Arg Ser
45                  50
CTATTAGGGT CCCCATTCTA CAGATGAGGA AACTGAA 232

SEQ ID NO:15
LENGTH:196
TYPE: nucleic acid
STRANDEDNESS: double
TOPOLOGY: linear
MOLECULE TYPE:cDNA to mRNA
ORIGINAL SOURCE:
ORGANISM:human
CLONE:981
FEATURE:
KEY: CDS
LOCATION: 67.. 102
IDENTIFICATION METHOD: E
SEQUENCE
CAAGTTCAGA TTAGAGAGGC CACTTTCCCA GAATCCACAG CTGCACTAAG NTAAGNGAGA 60

AGCCAG ATG CCG TTT TAC TGN GGT GTC AGG GGG CTG TTC TGAAGCTTGG 109
Met Pro Phe Tyr Tyr Gly Val Arg Gly Leu Phe
1               5                   10
GGGGGNTCAT TTTGAAAGGC CTTTCTTTCC CCTGGGGNAC CTNGGNTACC TTGGGGGACC 169
TACAAAGGTN GGTTGAGGGG AAGGGTG 196

SEQ ID NO:16
LENGTH:522
TYPE: nucleic acid
STRANDEDNESS: double
TOPOLOGY: linear
MOLECULE TYPE:cDNA to mRNA
ORIGINAL SOURCE:
ORGANISM:human
CLONE:1984
FEATURE:
KEY: CDS
LOCATION: 37.. 198
IDENTIFICATION METHOD: E
SEQUENCE
GAATTCGGCA CGAGCAGCTG CAGCAGAAGA GCGGAA ATG TCA AAC GTG GAG AGC 54
Met Ser Asn Val Glu Ser
1               5
CTA AAG ATA GTA ATG AGC AGC TTG AGT CTT AAG TTT TTG AAT ACA CCG 102
Leu Lys Ile Val Met Ser Ser Leu Ser Leu Lys Phe Leu Asn Thr Pro
            10                  15                  20
ATG GAA GTT GAC AAA GAT TCT TTG CTT GGC CAA ACT TTA GTC AGA CTC 150
Met Glu Val Asp Lys Asp Ser Leu Leu Gly Gln Thr Leu Val Arg Leu
        25                  30                  35
CTC AAC CTT CTC GTA GGT TTA TCT GTG CAT TTC CTT GGA AAA TCC AGT 198
Leu Asn Leu Leu Val Gly Leu Ser Val His Phe Leu Gly Lys Ser Ser
    40                  45                  50
TAGAACCCTC TACCCTCAAT ATCTGATCAG GTTCCTCATC CTCTACCATC CAAGGTGATG 258
TCTGATCACC TTGACCTATT TTCAGCAAGA AACCCCTTTA CTCCTGAAGT TCCTTCTTAG 318
TAATTTTTTT ATCCAGTGAC CCCAAGCTTT TTGTTCCTTG GCTATAAATT CCCACTTGCC 378
CATGCTGTAT TTAGAGTTGA GCCCACTCTC TCTCTCCTAA TTCAGAATCC CATTGCCATG 438
GTTGCTATAC CTATCCTGAT GGTCCGGAAT AAAATCTGTC TTACTGTGCT TTAAAAAAAA 498
AAAAAAAAAA AAAAAAAAAA AAAA 522

SEQ ID NO:17
LENGTH:1908
TYPE: nucleic acid
STRANDEDNESS: double
TOPOLOGY: linear
MOLECULE TYPE:cDNA to mRNA
ORIGINAL SOURCE:
  ORGANISM:human
  CLONE:2593
FEATURE:
  KEY: CDS
  LOCATION: 640.. 1002
  IDENTIFICATION METHOD: E
SEQUENCE
GAATTCGGCA CGAGGATCTC GCCGCGGTTC CGCGGCCCTG CCGCCGCCGC CGCCAGCAGA 60
GCGCACCGGG CCGATCGGGC GAGTGGCCAT GGCGGCGCCC GAGGACTGGC CGGGCCAGCA 120
GCTGGAGCTG GACGAGGACG AGGCGTCTTG TTGCCGCTGG GGCGCGCAGC ACGCCGGGGC 180
CCGCGAGCTG GCTGCGCTCT ACTCGCCAGT GCCCTTCACT CTCTGAAGAA CAGCCATGCC 240
TGAATCAGAG TCTGCAGACA GCCTGGTGCC CCAAATCTGC AATCAAGGCC CTTTATCTCC 300
TCCAGACCTG GAGGCAAGCG CCTCCAGGAG TGGTGCTCTG TGATCCTGTG CTTCAGCCTC 360
ATCGCCCACA ACCTGGTCCA TCTCCTGCTG CTGGCCCGCT GGGAGGACAC ACCCCTCGTC 420
ATACTCGGTG TTGTTGCAGG GGCTCTCATT GCTGACTTCT TGTCTGGCCT GGTACACTGG 480
GGTGCTGACA CATGGGGCTC TGTGGAGCTG CCCATTGTGG GGAAGGCTTT CATCCGACCC 540
TTCCGGGAGC ACCACATTGA CCCGACAGCT ATCACACGGC ACGACTTCAT CGAGACCAAC 600
GGGGACAACT GCCTGGTGAC ACTGCTGCCG CTGCTAAAC ATG GCC TAC AAG TTC 654
Met Ala Tyr Lys Phe
1               5
CGC ACC CAC AGC CCT GAA GCC CTG GAG CAG CTA TAC CCC TGG GAG TGC 702
Arg Thr His Ser Pro Glu Ala Leu Glu Gln Leu Tyr Pro Trp Glu Cys
                10                  15                  20
TTC GTC TTC TGC CTG ATC ATC TTC GGC ACC TTC ACC AAC CAG ATC CAC 750
Phe Val Phe Cys Leu Ile Ile Phe Gly Thr Phe Thr Asn Gln Ile His
            25                  30                  35
AAG TGG TCG CAC ACG TAC TTT GGG CTG CCA CGC TGG GTC ACC CTC CTG 798
Lys Trp Ser His Thr Tyr Phe Gly Leu Pro Arg Trp Val Thr Leu Leu
        40                  45                  50
CAG GAC TGG CAT GTC ATC CTG CCA CGT AAA CAC CAT CGC ATC CAC CAC 846
Gln Asp Trp His Val Ile Leu Pro Arg Lys His His Arg Ile His His
    55                  60                  65

GTC TCA CCC CAC GAG ACC TAC TTC TGC ATC ACC ACA GGC TGG CTC AAC 894
Val Ser Pro His Glu Thr Tyr Phe Cys Ile Thr Thr Gly Trp Leu Asn
70                  75                  80                  85
TAC CCT CTG GAG AAG ATA GGC TTC TGG CGA CGC CTG GAG GAC CTC ATC 942
Tyr Pro Leu Glu Lys Ile Gly Phe Trp Arg Arg Leu Glu Asp Leu Ile
                90                  95                  100
CAG GGC CTG ACG GGC GAG AAG CCT CGG GCA GAT GAC ATG AAA TGG GCC 990
Gln Gly Leu Thr Gly Glu Lys Pro Arg Ala Asp Asp Met Lys Trp Ala
            105                 110                 115
CAG AAG ATC AAA TAACTTCTCC GAGCCTGCTA CCTGGTTGCC AACCTTCCCT 1042
Gln Lys Ile Lys
        120
AGCCCCCAAA CCGAAGCCAT CTGCCAAATT CCAGCCTCTT TGAGCTGGCC CCTCCAGATG 1102
GAGAGGACAT CTCCTGGGCT GGGCCCAGGT ACCCCAGCCC ACCCCTCATG ACACAGAATA 1162
CTTGAGCCAC TGATTTTTCA TTTCTTTTTT TTTTTTTTCC TCGGCCCCTC CTCAGCCACC 1222
TGAGTTGCTC TATCTGCAAG CCTGACTCTG CCAGCCTCCC CTGGTAGAGA GGAGGTTTAC 1282
CCACTCCCTG CACGCCTGCC GTCCCTGCCC CGCTGGGCAG CCCTTCAGTG TGGCTGGCGT 1342
TGGGGCCAGT GAGTTGCCTC TTTCCCTCCT TGTCTGGCCC CAGTGGTCTG GGGAGCCCCC 1402
AGGCACACCT AAGCGTCGTG GAGCATTGTT CTGCCACAGC CCTGCATACT GACCCCGGGA 1462
GGCTGGGCAG GTGGACAGCC CCAGCCACCA CCTTCAGCCT AGCCTGTCCC CCAAGGATGG 1522
TGAAGCTCAG CAGGGGTCTG AGGGTAGCCG GCCAGAAGAG GCTGGAACCT CCTGCTCAAG 1582
TCTAGACCCC TACTTCTCTG CTGCCCCCAC CCTGCCAGAG CTGATGTTTC CAATACCAAG 1642
ATGTCTTCAC AGGGCACAGC CCCTGCAGAG CATCTTGGTC ATTTGGAAGA GGACACGGTA 1702
TCCCCTCTGG CCAGAGTATG TCAGAGAAGG AAGAGTAGGG CTTTTTTGTT TTGTTTTTTT 1762
TTAAAGGTGC TTGCTTGTTT AATGTAAATA ATAGAAAGCC TTAATATCTT TTCTGTAACA 1822
CGGAGTAATA TTTTAATGTC ATGTTTTGGA TGTACATAAT ATATTTATAA CAAAGAAAAA 1882
AAAAAAAAAA AAAAAAAAAA AAAAAA 1908

## Claims

1. A DNA having a nucleotide sequence complementary to the RNA strand of mRNA, the expression level of which varies in the brain of a patient with Alzheimer's disease, compared with the expression level of the mRNA in normal human brain.

2. The DNA according to claim 1, wherein the DNA has a complementary sequence to the RNA strand of mRNA, the expression level of which is increased by two-fold or more.

3. The DNA according to claim 2, wherein the DNA has the nucleotide sequence selected from Sequence Nos.1, 2, 3, 4, 5, 6, 7, 8, 9, 10, 11, 12, 13, 14 and 15.

4. The DNA according to claim 1, wherein the DNA has a complementary sequence to the RNA strand of mRNA, the expression level of which is decreased by 0.5-fold or less.

5. The DNA according to claim 4, wherein the DNA has the nucleotide sequence selected from Sequence Nos.16 and 17.

6. A DNA wherein one or more nucleotides in a DNA selected from DNAs according to claims 1, 2, 3, 4 and 5 are deleted or substituted with other nucleotides or one or more nucleotides are added to said DNA and/or which is hybridizable with the DNA selected from DNAs according to claims 1, 2, 3, 4 and 5 under stringent conditions.

7. A polypeptide encoded by a DNA selected from DNAs according to claims 1, 2, 3, 4, 5 and 6.

8. An antibody binding to a polypeptide according to claim 7.

9. A diagnostic agent of patients with Alzheimer's disease, containing a DNA selected from DNAs having a nucleotide sequence selected from Sequence Nos.1, 2, 3, 4, 5, 6, 7, 8, 9, 10, 11, 12, 13, 14, 15, 16 and 17.

10. A therapeutic agent of patients with Alzheimer's disease, containing a DNA having the nucleotide sequence selected from Sequence Nos.1, 2, 3, 4, 5, 6, 7, 8, 9, 10, 11, 12, 13, 14, 15, 16 and 17.

11. A therapeutic agent of patients with Alzheimer's disease, containing a polypeptide according to claim 7.

12. A diagnostic agent of patients with Alzheimer's disease, containing an antibody according to claim 8.

13. A therapeutic agent of patients with Alzheimer's disease, containing an antibody according to claim 8.
